# EUROPEAN PATENT SPECIFICATION

(11) **EP 2 147 105 B1**
(45) Date of publication and mention of the grant of the patent: **24.04.2013**
(21) Application number: 07776563.4
(22) Date of filing: 02.05.2007
(51) Int. Cl.: C12N 15/63, C12N 15/68, C12N 15/64, C12N 15/85

(54) **DNA PLASMIDS HAVING IMPROVED EXPRESSION AND STABILITY**
DNA-PLASMIDE MIT VERBESSERTER EXPRESSION UND STABILITÄT
PLASMIDES À ADN À EXPRESSION ET STABILITÉ AMÉLIORÉES

(43) Date of publication of application: 27.01.2010
(73) Proprietor: Merial Limited, Duluth, GA 30096 (US)
(72) Inventor: AUDONNET, Jean-christophe, F-69006 Lyon (FR)
(74) Representative: Harding, Charles Thomas
(86) International application number: PCT/US2007/010547
(87) International publication number: WO 2008/136790

(56) References cited:
- WO-A-2005/024015
- US-A- 5 116 750
- US-A- 5 256 568
- US-A- 5 538 868

## Description

### FIELD OF THE INVENTION

The present description relates generally to DNA vaccines and methods of using the same. More particularly, the present invention relates to DNA plasmids with improved expression and stability useful for DNA vaccines.

### BACKGROUND OF THE INVENTION

DNA vaccines, also referred to as genetic, plasmid or polynucleotide vaccines, represent a relatively simple and economical method to exploit gene transfer for immunization against antigens. The low toxicity associated with DNA vaccines favors its further development, but additional strategies to improve the potency of this approach are needed if it is to be successfully integrated into the clinical setting (reviewed by Shaw & Strong, Front Biosci. 2006 Jan 1;11:1189-98). DNA vaccination can overcome most disadvantages of conventional vaccine strategies and has potential for vaccines of the future. However, a commercial product still has not reached the market. One possible explanation could be the technique's failure to induce an efficient immune response in humans, but safety may also be a fundamental issue (reviewed by Glenting & Wessels, Microb Cell Fact. 2005 Sep 6;4:26).

The pVR1020 or 1012 plasmid (VICAL Inc.; Luke C. et al., Journal of Infectious Diseases, 1997, 175, 91-97; Hartikka J. et al., Human Gene Therapy, 1996, 7,1205-1217, see, e.g., U.S. Patent Nos. 5,846,946; 6,451,769; 6,586,409 and 6,875,748) is a DNA plasmid vector utilized for the insertion of a polynucleotide sequence. The pVR1020 plasmid is derived from pVR1012 and contains the human tPA signal sequence. Additional DNA plasmids are found, for example, in U.S. Patent Nos. 6,852,705; 6,818,628; 6,586,412; 6,576,243; 6,558,674; 6,464,984; 6,451,770; 6,376,473 and 6,221,362. However, there are disadvantages with the pVR1012-based plasmids for DNA vaccination, such as plasmid instability and copy-number heterogeneity.

Accordingly, there is a need for an effective DNA vaccine, especially with respect to expression of a target antigen, epitope, immunogen, peptide or polypeptide of interest in an amount sufficient to elicit a protective response.

### SUMMARY OF THE INVENTION

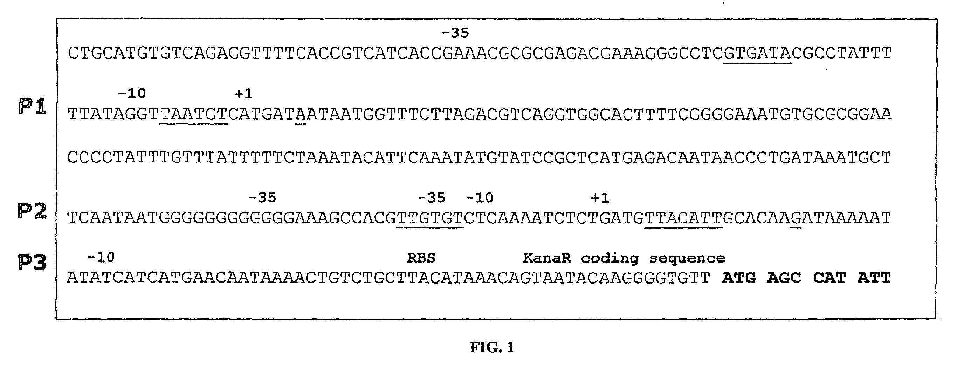

The present invention provides a DNA plasmid comprising a kanamycin resistance gene comprising a kanamycin resistance gene promoter wherein the kanamycin resistance gene promoter consists of the P1 promoter of Figure 1, wherein the expression of the kanamycin resistance gene is decreased in said DNA plasmid as compared to expression of the kanamycin resistance gene in the plasmid pVR1012 and wherein the decreased kanamycin resistance gene expression results in higher plasmid yields and higher plasmid stability.

In another aspect, the present invention provides a formulation for delivery and expression of an antigen, epitope, immunogen, peptide or polypeptide of interest, wherein the formulation comprises the plasmid of the present invention and a pharmaceutically or veterinarily acceptable carrier, vehicle or excipient.

In a further aspect, the present invention provides a kit for stimulating an immune response in an animal or eliciting an immune response in an animal wherein said kit comprises the DNA plasmid of the present invention or the formulation of the present invention and instructions for stimulating an immune response in an animal or eliciting an immune response in an animal.

The invention is based, in part, on an experimental observation that insertion of a transposon between a kanamycin resistance gene promoter and translation initiation abolished kanamycin resistance, as expected, and unexpectedly, enabled high rates of plasmid replication. In other words, plasmid yields of the mutated form (containing the transposon) were three times higher than the parental plasmid yields. It was therefore hypothesized that expression efficiency of the kanamycin resistance ("KanaR") gene has an impact on plasmid replication in bacteria, i.e., decreased kanamycin resistance expression leads to increased plasmid replication rates. Specifically, the high KanaR expression rate may represent a strong metabolic burden, which interferes with optimal plasmid replication rate and/or KanaR gene transcription interferes with the replication origin (ORI).

The present description relates to a DNA plasmid which may comprise a kanamycin resistance ("KanaR") gene wherein the kanamycin resistance gene comprises a less effective promoter for KanaR expression and/or a less efficient initiation codon. In an advantageous embodiment, the KanaR promoter is the P1 promoter. The resultant DNA plasmid results in higher plasmid yields and higher plasmid stability presumably due to decreased KanaR expression. Advantageously, the DNA plasmid may be pLL10 or pLL14.

The present description also relates to a DNA plasmid which may comprise a kanamycin resistance gene wherein a transposon is inserted between the KanaR promoter and translation initiation, thereby resulting in decreased KanaR expression. The resultant DNA plasmid results in higher plasmid yields and higher plasmid stability presumably due to decreased KanaR expression.

The present description encompasses formulations for delivery and expression of an antigen, epitope, immunogen, peptide or polypeptide of interest, wherein the formulation may comprises any of the DNA plasmids disclosed herein and a pharmaceutically or veterinarily acceptable carrier, vehicle or excipient In an advantageous embodiment, the carrier, vehicle or excipient may facilitate transfection and/or improves preservation of the vector or protein. Advantageously, the antigen, epitope, immunogen, peptide or polypeptide of interest may be derived from an avian, bovine, canine, equine, feline or porcine virus or pathogen.

The description further provides for methods of stimulating an immune response in an animal which may comprise administering an effective amount of the formulations disclosed herein to cells of the animal and expressing the antigen, epitope, immunogen, peptide or polypeptide of interest in the cells. The description also provides for methods of eliciting an immune response in an animal which may comprise administering an effective amount of the formulations disclosed herein to cells of the animal and expressing the antigen, epitope, immunogen, peptide or polypeptide of interest in the cells. Advantageously, the animal may be an avian, a bovine, a canine, an equine, a feline or a porcine.

The description also encompasses kits for performing any one of the methods of described above which may comprise the DNA plasmid or formulations of disclosed herein plus instructions for performing the methods of stimulating or eliciting an immune response in an animal.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings, in which:
**FIG. 1** depicts the three potential promoters of the KanaR expression cassette as identified in Example 1: P1 (SEQ ID NO: 1), P2 (SEQ ID NO: 2) and P3 (SEQ ID NO: 3). P2 and P3 partially overlap;
**FIG. 2** depicts a schematic of pVR1012, illustrating the direction of KanaR transcription and the position of the replication origin, ORI;
**FIG. 3** illustrates how the KanaR cassette of plasmid pVR1012 in Example 1 was cloned into the mutagenesis vector pALTER-1;
**FIG. 4** depicts how a *PacI* restriction site in Example 1 was introduced downstream of the KanaR ORF, producing plasmid pLL2, a *SwaI* restriction site was introduced upstream, a RsrII restriction site was introduced downstream, producing pLL4, and finally the translation initiator codon ATG was mutated to TTG, producing pLL6;
**FIG. 5** depicts how the mutated KanaR cassette of pLL4 in Example 1 was cloned into plasmid pVR1012, producing pLL7;
**FIG. 6** depicts how the rmB terminator sequence identified in Example 1 was cloned into plasmid pLL7 by PCR from pSB062 and subsequent *PacI RsrII* digestion to form pLL9;
**FIG. 7** depicts the cloning of the speA terminator sequence, identified in Example 1, into plasmid pLL7 by annealing of synthetic oligonucleotides and *PacI RsrII* digestion to form pLL11;
**FIG. 8** depicts how the mutated translation initiation sequence of plasmid pLL6 was cloned into pLL9 by *MscI PacI* digestion to form pLL10;
**FIG. 9** illustrates how the promoter sequences P2/P3 (SEQ ID NOS: 2-3) with P1 (SEQ ID NO: 1), P1 (SEQ ID NO: 1), and P2/P3 (SEQ ID NOS: 2-3), which were identified in Example 1, were cloned by URS11-URS12 PCR, URS13-URS12 PCR and URS11-URS14 PCR, respectively;
**FIG. 10** depicts how promoter sequence P2/P3 (SEQ ID NOS: 2-3) with P1 (SEQ ID NO: 1) was cloned into plasmid pLL9 to form pLL13;
**FIG. 11** depicts how promoter sequence P1 (SEQ ID NO: 1) was cloned into plasmid pLL9 to form pLL14;
**FIG. 12** depicts how promoter sequence P2/P3 (SEQ ID NOS: 2-3) was cloned into plasmid pLL9 to form pLL15;
**FIG. 13** depicts how promoter sequence P1 (SEQ ID NO: 1) was cloned into plasmid pLL7 to form pLL16;
**FIG. 14** depicts several pVR1012 derivatives generated with the various suitable promoters and terminators identified in Example 1. The pLL13, pLL14, pLL15 and pLL16 constructs have a 192 base pair deletion between the CMV promoter and the Kana P1 (SEQ ID NO: 1) promoter;
**FIG. 15** depicts a schematic of pVR1012 derivatives, illustrating the 192 base pair region deleted in the pLL13 to pLL16 constructs of Example 1;
**FIG. 16** depicts two graphs of the ability of bacterial colonies to resist increasing Kanamycin concentrations, according to Example 1, at 50 µl and 100 µl bacterial suspension dilutions, respectively;
**FIG. 17** depicts graphs of the plasmid yields obtained under laboratory growth conditions. The bacterial colonies of Example 1 were grown in liquid LB medium with Kana 100 µg/ml;

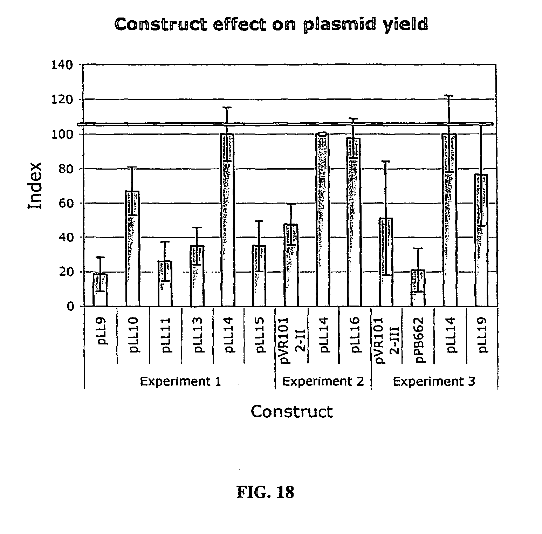

**FIG. 18** depicts plasmid yields expressed as a ratio to the pLL14 plasmid yield (baseline =100). Experiment 1 depicts 8 to 15 clones per construct, Experiment 2 depicts 6 clones for pVR1012, 2 for pLL14 and 25 for pLL16 and Experiment 3 (25 clones for pVR1012, pPB662 and pLL14, and 20 for pLL19);
**FIG. 19** depicts graphs of the plasmid yields obtained under laboratory growth conditions. The bacterial colonies were grown in EGLI medium. Plasmid yields are expressed as a ratio to the pVR1012 parental plasmid yield (baseline = 100) and
**FIG. 20** depicts individual plasmid yield (indexed on pVR1012 construct average) showing clone heterogeneity. (*: mutated plasmid) and depicts graphs of the plasmid yields in three different construct EGLI-adapted clones according to Example 1. Variability is expressed as an index, where the first clone is considered the 100% reference.

### DETAILED DESCRIPTION

The invention is based, in part, on an experimental observation that insertion of a transposon between a kanamycin resistance gene promoter and translation initiation abolished kanamycin resistance, as expected, and unexpectedly, enabled high rates of plasmid replication. In other words, plasmid yields of the mutated form (containing the transposon) were three times higher than the parental plasmid yields. It was therefore hypothesized that expression efficiency of the kanamycin resistance ("KanaR") gene has an impact on plasmid replication in bacteria, i.e., decreased kanamycin resistance expression leads to increased plasmid replication rates. Specifically, in the context of nutrient limitation, the high KanaR expression rate may represent a strong metabolic burden, which interferes with optimal plasmid replication rate and/or KanaR gene transcription interferes with the replication origin (ORI).

The description encompasses any contemplated DNA plasmid wherein KanaR expression is decreased, thereby resulting in increased plasmid yields and higher plasmid stability as compared to, for example, the pVR1012 plasmid or a derivative thereof, without reduced KanaR expression.

The present description relates to a DNA plasmid which may comprise KanaR wherein the kanamycin resistance gene comprises a less effective promoter for KanaR expression and/or a less efficient initiation codon. In an advantageous embodiment, the KanaR promoter is the P1 promoter and the initiation codon is ATG. The resultant DNA plasmid results in higher plasmid yields and higher plasmid stability presumably due to decreased KanaR expression. Advantageously, the DNA plasmid may be pLL10 or pLL14.

The present description also relates to a DNA plasmid which may comprise a kanamycin resistance gene wherein a transposon is inserted between the KanaR promoter and translation initiation, thereby resulting in decreased KanaR expression. The resultant DNA plasmid results in higher plasmid yields and higher plasmid stability presumably due to decreased KanaR expression.

The expression vector is a plasmid vector or a DNA plasmid vector, in particular an *in vivo* expression vector. In a specific, non-limiting example, the DNA plasmid vector contains elements of the pVR1020 or 1012 plasmid (VICAL Inc.; Luke C. et al., Journal of Infectious Diseases, 1997, 175, 91-97; Hartikka J. et al., Human Gene Therapy, 1996, 7, 1205-1217, see, e.g., U.S. Patent Nos. 5,846,946; 6,451,769; 6,586,409 and 6,875,748). The pVR1020 plasmid is derived from pVR1012 and contains the human tPA signal sequence. In one embodiment the human tPA signal comprises from amino acid M(1) to amino acid S(23) in Genbank under the accession number HUMTPA14. In another specific, non-limiting example, the plasmid utilized as a vector for the insertion of a polynucleotide sequence can contain the signal peptide sequence of equine IGF1 from amino acid M(24) to amino acid A(48) in Genbank under the accession number U28070. Additional information on DNA plasmids which may be consulted or employed in the practice are found, for example, in U.S. Patent Nos. 6,852,705; 6,818,628; 6,586,412; 6,576,243; 6,558,674; 6,464,984; 6,451,770; 6,376,473 and 6,221,362.

In another embodiment, the plasmid may be any plasmid with a kanamycin resistance gene including, but not limited to, Clontech vectors Living Colors pAcGFP1, Living Colors pAcGTP1-C1, Living Colors pAcGFP1-N1, pAmCyan1-C1, pAmCyan1-N1, pAsRed2-C1, pAsRed2-N1, pCMV-DsRed-Express, pDsRed2-1, pDsRed2-C1, pDsRed2-N1, pDsRed-Express-I, pBsRed-Express-C1, pDsRed-Express-N1, pDsRed-Monomer-C1, pDsRed-Monomer-N1, pHcRed1-1, pHcRed1-C1, pHcRed1-N1/1, pIRES2-DsRed2, pIRES2-DsRed-Express, pLPS-AcGFP1-N Acceptor, Proteasome Sensor, pTimer, pZsGreen1-1, pZsGreen1-C1, pZsGreen1-N1, pZsYellow1-C1 and pZsYellow1-N1 Vector; Invitrogen vectors pCR3, pCR3.1-Uni, PCR1000 and pZErO-2.1 and Stratagene pCMV-3Tag, pBK-CMV Phagemid, ZAP Express, pCMV-Script, pCMV-Tag, PCR-Script pMC1neo and pMC1neo Poly A vectors. Reference is also made to U.S. Patent Nos. 6,942,975, 6,887,702, 6,849,442, 6,846,970, 6,825,012, 6,806,399, 6,803,230, 6,790,607, 6,696,278, 6,667,150,6,624,344, 6,620,990, 6,610,909, 6,585,976, 6,573,437, 6,570,067, 6,562,584, 6,528,703, 6,503,748, 6,475,731, 6,410,317, 6,410,314, 6,387,654, 6,376,744, 6,350,934, 6,303,383, 6,297,054, 6,284,541, 6,258,999, 6,255,560,6,248,937, 6,235,518, 6,174,708, 6,127,171, 6,121,511, 6,117,651, 6,054,635, 6,037,524, 6,018,103, 5,994,625, 5,981,191, 5,981,182, 5,977,439, 5,925,544, 5,910,488, 5,866,404, 5,851,808, 5,851,804, 5,830,727, 5,824,877, 5,792,935, 5,783,394, 5,750,871, 5,733,753, 5,733,744, 5,731,179, 5,723,746, 5,716,803,5,712,112, 5,705,361, 5,654,180, 5,599,670,5,591,577, 5,589,623, 5,569,834, 5,567,599, 5,565,347, 5,504,005, 5,463,174, 5,460,952, 5,436,138, 5,416,250, 5,416,011, 5,378,618, 5,256,568, 5,169,755, 5,137,829, 5,053,335, 5,004,863, 4,935,340, 4,920,054, 4,795,855,4,782,022, 4,771,002, 4,626,510, and 4,567,146.

The present description also encompasses plasmids with other antibiotic resistance genes in addition to kanamycin resistance, such as but not limited to ampicillin, chloramphenicol, neomycin and tetracycline resistance.

In one embodiment, the plasmid may be any plasmid with an ampicillin resistance gene including, but not limited to, Clontech vectors Living Colors pAcGFP1, pAmCyan, pAsRed2, pbetagal-Basic, pbetagal-Control, pBI Tet, pBI-G Tet, pBI-L Tet, pCMVbeta, pCMV-Myc & pCMV-HA, pDsRed2, pDsRed-Express, pDsRed-Monomer Vector, pGFP, pGFPuv, pHcRed1, pIRES, pIRESbleo3,, pIRESneo3, pIKESpuro3, pLP-IRESneo Acceptor, pLP-LNCX Acceptor, pLP-TRE2 Acceptor, , pPUR, pRevTet-Off, pRevTet-Off-IN, pRevTet-On, pSEAP2-Basic, pSEAP2-Control, pTet-Off, pTet-On, pTet-tTS, pTimer, pTimer-1, pTK-Hyg, pTRE2 , pTRE2hyg, pTRE2hyg2-6xHN, pTRE2hyg2-HA, pTRE2hyg2-Myc, pTRE2pur, pTRE2pur-6xHN, pTRE2pur-HA, pTRE2pur-Myc, pTRE-6xHN, pTRE-HA, pTRE-Myc, pTRE-Tight, pTRE-Tight-DsRed2, pZsGreen and pZsYellow; Invitrogen vectors p2Bac, p2Bac/CAT/CAT, pAc5.1/V5-His, pAc5.1/V5-His/LacZ, pBAD-TOPO, pBlueBac, pBlueBac/His, pBlueBac/His/CAT, pBlueBac2, pBlueBac3, pBlueBac4, pBlueBac4/CAT, pcDNA1/Amp, pcDNA3/CAT, pcDNAII, pCMVSport1, pCMVSport2, pCMVSport2.2, pCMVSport4, pCR3, pCR3.1-Uni, pCRBac, pCRT7/CT-LacZ, pCRT7/CT-TOPO, pCRT7/NT-E3, pCRT7/NT-TOPO, pDW232, pFastBac1, pMEP4, pMT/LacZ, pMT/V5-His-TOPO, pPIC3, pPIC3K, pRBK, pSL301, pSPORT1-CAT, pYesTrp2-RaIGDS and pYesTrp2-Ra1GDS and Stratagene PCR-Script, pBC Phagemid, pMC1neo and pMC1neo Poly A, pBlueScript II Phagemid and SuperCos I vectors. Reference is also made to U.S. Patent Nos. 6,987,006, 6,972,322, 6,916,611, 6,887,702, 6,803,230, 6,790,607, 6,706,524, 6,696,278, 6,667,150, 6,569,678, 6,562,584, 6,544,782, 6,521,449, 6,503,748,6,486,134, 6,410,317, 6,410,314, 6,387,654, 6,376,192, 6,291,238, 6,255,071, 6,221,630, 6,140,087, 6,127,171, 6,025,193, 6,025,192, 6,010,875, 5,981,279, 5,981,182, 5,955,363,5,925,544, 5,919,676, 5,912,153, 5,894,060, 5,877,400, 5,866,404, 5,851,808, 5,834,191, 5,830,727, 5,830,690, 5,786,162, 5,776,773, 5,773,697, 5,766,940, 5,733,753, 5,731,193, 5,716,803, 5,705,361, 5,691,155, 5,602,300, 5,527,691, 5,504,005, 5,470,729, 5,436,138, 5,434,065, 5,432,082, 5,378,618, 5,290,691, 5,264,354, 5,256,568, 5,256,546, 5,160,489, 5,151,364, 5,147,789, 5,143,836, 5,126,252, 5,093,251, 5,081,022, 4,997,767, 4,988,622, 4,935,364, 4,920,054, 4,889,806, 4,879,230,4,876,202, 4,845,031, 4,808,519, 4,766,072, 4,752,574, 4,703,012, 4,634,678,487,835, 4,349,629 and 4,340,674.

In another embodiment, the plasmid may be any plasmid with a chloramphenicol resistance gene including, but not limitd to, Clontech vectors pDNR-LacZ Donor Reporter, pDNR-SEAP Donor Reporter, pLP-AcGFP1-C Acceptor, pLP-BacPAK9 Acceptor, pLP-BacPAK9-6xHN Acceptor, pLP-CMV-HA Acceptor, pLP-CMV-Myc Acceptor, pLP-CMVneo Acceptor, pLP-GADT7 AD Acceptor, pLP-GBKT7 DNA-Acceptor, pLP-IRESneo Acceptor, pLP-LNCX Acceptor, pLP-PROTet-6xHN Acceptor, pLP-RevTRE Acceptor, pLPS-AcGFP1-N Acceptor and pLP-TRE2 Acceptor; Invitrogen vectors pLysS and pSPORT1-CAT and Stratagene pBC Phagemid and PCR-Script vectors. Reference is also made to U.S. Patent Nos. 6,916,611, 6,900,010, 6,887,702, 6,884,576, 6,846,671, 6,803,230, 6,696,278, 6,673,537, 6,667,150, 6,562,584, 6,548,246, 6,503,748, 6,436,694, 6,420,110, 6,410,317, 6,410,314, 6,391,640, 6,387,654, 6,376,192, 6,331,527, 6,309,883, 6,309,830, 6,291,211, 6,255,071, 6,252,140, 6,221,630, 6,146,871, 6,127,171, 6,107,093, 6,083,750, 6,031,151, 6,025,192, 6,001,564, 5,994,132, 5,994,066, 5,981,182, 5,955,363, 5,932,479, 5,928,891, 5,925,544, 5,925,523, 5,908,747, 5,866,404, 5,851,808, 5,821,093, 5,766,940, 5,733,753, 5,728,571, 5,716,803, 5,707,830, 5,639,644, 5,591,577, 5,470,729, 5,437,988, 5,434,065, 5,256,568, 5,256,546, 5,053,335, 5,004,863, 4,868,111, 4,839,284, 4,752,574 and 4,711,849.

In another embodiment, the plasmid may be any plasmid with a neomycin resistance gene including, but not limited to, Clontech vectors pRevTet-Off Vector, pRevTet-Off-IN Vector, pIRES Vector, pIRESbleo3 Vector, pIREShyg3 Vector, pIRESneo3 Vector, pIRESpuro3 Vector, pLP-IRESneo Acceptor Vector, pTet-Off Vector, pTet-On Vector, pIRES2-DsRed-Express Vector, pLXIN Retroviral Vector, pIRES2-DsRed2 Vector, Proteasome Sensor Vector, pLXSN Retroviral Vector, pCMV-DsRed-Express Vector, Living Colors pAcGFP1-C1 Vector, pAmCyan1-C1 Vector, pAsRed2-C1 Vector, pDsRed2-C1 Vector, pDsRed-Express-C1 Vector, pDsRed-Monomer-C1 Vector, pHcRed1-C1 Vector, pZsGreen1-C1 Vector, pZsYellow1-C1 Vector, Living Colors pAcGFP1 Vector, pDsRed2-1 Vector, pDsRed-Express-1 Vector, pHeRed1-1 Vector, pTimer Vector, pZsGreen1-1 Vector, pQCXIX Retroviral Vector, LRCX Retroviral Vector Set, pLNCX2 Retroviral Vector, pLP-LNCX Acceptor Vector, pQCXIN Retroviral Vector, Living Colors pAcGFP1-N1 Vector, pAmCyan1-N1 Vector, pAsRed2-N1 Vector, pDsRed2-N1 Vector, pDsRed-Express-N1 Vector, pDsRed-Monomer-N1 Vector, pHcRed1-N1/1 Vector, pLPS-AcGFP1-N Acceptor Vector, pZsGreen1-N1 Vector, pZsYellow1-N1 Vector and VP16 Minimal Domain Vector Set; Invitrogen vectors pcDNA1/Neo, pcDNA3/CAT (replaced with pcDNA3.1/CAT), pCR3 (replaced with pCR3.1), pCR3.1-Uni and pRc/CMV and Stratagene pCMV-3Tag Vectors, pCMV-Script Vector, SuperCos I Vector, ZAP Express Vector, pCMV-Tag Vectors, pMC1neo and pMC1neo Poly A Vectors, PCR-Script Cloning Kits, Vitality hrGFP Mammalian Expression Vectors, pBK-CMV Phagemid Vector and Lambda ZAP-CMV Vector. Reference is also made to U.S. Patent Nos. 7,026,525, 6,942,995, 6,905,818, 6,815,185, 6,806,399, 6,787,687, 6,747,189, 6,673,602, 6,673,537, 6,667,150, 6,624,344, 6,620,990, 6,440,444, 6,429,357, 6,391,633, 6,376,192, 6,316,253, 6,294,187, 6,291,211, 6,255,560, 6,252,140, 6,248,937, 6,232,526, 6,221,630, 6,210,930, 6,207,879, 6,194,636, 6,114,146, 6,096,717, 6,071,512, 5,998,144, 5,985,560, 5,969,211, 5,955,363, 5,955,319, 5,939,288, 5,902,577, 5,894,060, 5,891,634, 5,830,698, 5,807,742, 5,750,871, 5,733,779, 5,665,565, 5,639,663, 5,614,381, 5,583,278, 5,470,726, 5,463,174, 5,416,011, 5,352,605, 5,278,056, 5,256,568, 5,149,636, 5,017,478, 4,957,865, 4,935,340, 4,839,284, 4,792,520, 4,766,066, 4,752,574, 4,740,463, 4,663,285, 4,536,475, 4,513,086, 4,513,085,4,503,155, 4,468,462, 4,460,688,4,430,434 and 4,416,994.

In another embodiment, the plasmid may be any plasmid with a tetracycline resistance gene including, but not limited to, Clontech vectors pRevTet-Off Vector, pRevTet-On Vector, pRevTet-Off-IN Vector, Creator-Compatible PROTet 6xHN Bacterial Expression System, PROTet 6xHN Bacterial Expression System, pTRE-Tight Vector, pTet-Off Vector, pTet-On Vector, Tet-Off Gene Expression System, Tet-On Gene Expression System, Adeno-X Tet-Off Expression System 1, Adeno-X Tet-On Expression System 1, Adeno-X Viral DNA (PI-Sce I/I-Ceu I digested), Creator-Compatible RevTet-Off Retroviral Gene Expression System, Creator-Compatible RevTet-On Retroviral Gene Expression System, pLP-RevTRE Acceptor Vector, pRevTRE Vector, RevTet-Off System, RevTet-On System, pBI Tet Vector, pBI-G Tet Vector, pBI-L Tet Vector, pTet-tTS Vector, pLP-TRE2 Acceptor Vector, pTRE2 Vector, pTRE2hyg Vector, pTRE2pur Vector, Tet System Approved FBS, US-Sourced (Gamma Irradiated), pTRE-Tight-DsRed2 Vector and VP16 Minimal Domain Vector Set and Invitrogen vectors pTet-tTak and pTet-Splice. Reference is also made to U.S. Patent Nos. 6,924,101, 6,905,836, 6,777,229, 6,759,236, 6,699,702, 6,699,692, 6,696,278, 6,680,301, 6,673,537, 6,667,150, 6,642,052, 6,620,618, 6,613,528, 6,544,782, 6,541,003, 6,503,748, 6,482,636, 6,440,741, 6,436,694, 6,410,317, 6,392,121, 6,376,192, 6,309,883, 6,303,383, 6,265,562, 6,261,807, 6,221,630, 6,218,181, 6,136,536, 6,127,171, 6,080,575, 6,033,856, 6,022,731, 6,010,875, 5,958,680, 5,955,363, 5,891,718, 5,869,035, 5,866,410, 5,858,762, 5,851,796, 5,849,576, 5,840,521, 5,830,727, 5,830,690, 5,786,162, 5,766,940, 5,637,503, 5,593,860, 5,585,254, 5,527,691, 5,516,669, 5,508,176, 5,384,259, 5,378,618, 5,348,886, 5,290,691, 5,256,568, 5,256,546, 5,166,070, 5,158,891, 5,151,364, 5,147,789, 5,053,335, 4,983,522, 4,879,230, 4,876,202, 4,874,703, 4,778,761, 4,752,574, 4,703,012, 4,680,260, 4,663,285, 4,634,677, 4,631,259, 4,581,335, 4,374,200 and 4,349,629.

The term plasmid covers any DNA transcription unit comprising a polynucleotide according to the description and the elements necessary for its *in vivo* expression in a cell or cells of the desired host or target; and, in this regard, it is noted that a supercoiled or non-supercoiled, circular plasmid, as well as a linear form, are intended to be within the scope of the description.

Each plasmid comprises or contains or consists essentially of, in addition to the polynucleotide encoding an antigen, epitope or immunogen, optionally fused with a heterologous peptide sequence, variant, analog or fragment, operably linked to a promoter or under the control of a promoter or dependent upon a promoter. In general, it is advantageous to employ a strong promoter functional in eukaryotic cells. The preferred strong promoter is the immediate early cytomegalovirus promoter (CMV-IE) of human or murine origin, or optionally having another origin such as the rat or guinea pig. The CMV-IE promoter can comprise the actual promoter part, which may or may not be associated with the enhancer part: Reference can be made to EP-A-260 148, EP-A-323 597, U.S. Patents Nos. 5,168,062, 5,385,839, and 4,968,615, as well as to PCT Application No WO87/03905. The CMV-IE promoter is advantageously a human CMV-IE (Boshart M. et al., Cell., 1985, 41, 521-530) or murine CMV-IE.

In more general terms, the promoter has either a viral or a cellular origin. A strong viral promoter other than CMV-IE that may be usefully employed in the practice of the description is the early/late promoter of the SV40 virus or the LTR promoter of the Rous sarcoma virus. A strong cellular promoter that may be usefully employed in the practice of the description is the promoter of a gene of the cytoskeleton, such as e.g. the desmin promoter (Kwissa M. et al., Vaccine, 2000, 18, 2337-2344), or the actin promoter (Miyazaki J. et al., Gene, 1989, 79, 269-277).

Functional sub fragments of these promoters, i.e., portions of these promoters that maintain an adequate promoting activity, are included within the present description, e.g. truncated CMV-IE promoters according to PCT Application No. WO98/00166 or U.S. Patent No. 6,156,567 can be used in the practice of the description. A promoter in the practice of the description consequently includes derivatives and sub fragments of a full-length promoter that maintain an adequate promoting activity and hence function as a promoter, preferably promoting activity substantially similar to that of the actual or full-length promoter from which the derivative or sub fragment is derived, e.g., akin to the activity of the truncated CMV-IE promoters of U.S. Patent No. 6,156,567 to the activity of full-length CMV-IE promoters. Thus, a CMV-IE promoter in the practice of the description can comprise or consist essentially of or consist of the promoter portion of the full-length promoter and/or the enhancer portion of the full-length promoter, as well as derivatives and sub fragments.

Preferably, the plasmids comprise or consist essentially of other expression control elements. It is particularly advantageous to incorporate stabilizing sequence(s), e.g., intron sequence(s), preferably the first intron of the hCMV-IE (PCT Application No. WO89/01036), the intron II of the rabbit β-globin gene (van Ooyen et al., Science, 1979, 206, 337-344).

As to the polyadenylation signal (polyA) for the plasmids and viral vectors other than poxviruses, use can more be made of the poly(A) signal of the bovine growth hormone (bGH) gene (*see* U.S. Patent No. 5,122,458), or the poly(A) signal of the rabbit β-globin gene or the poly(A) signal of the SV40 virus.

The description provides for the expression of any target peptide or polypeptide, advantageously an antigen, epitope, immunogen, peptide or polypeptide of interest by the DNA plasmids of the present invention. The description contemplates the expression of any antigen, epitope, immunogen, peptide or polypeptide of interest in the DNA plasmids disclosed herein. The DNA plasmids can express one or more antigens, epitopes or immunogens of interest

In an advantageous embodiment, the antigen, epitope, immunogen, peptide or polypeptide is derived from an avian, bovine, canine, equine, feline or porcine virus or pathogen. In another embodiment, the antigen, epitope, immunogen, peptide or polypeptide may be derived from West Nile virus. In another embodiment, the antigen, epitope, immunogen, peptide or polypeptide may be derived from a human virus or pathogen.

Avian antigens, epitopes or immunogens according to the description can be derived from Marek's disease virus (MDV) (e.g., serotypes 1 and 2, preferably 1), Newcastle disease virus (NDV), Gumboro disease virus or infectious bursal disease virus (IBDV), infectious bronchitis virus (IBV), infectious anaemia virus or chicken anemia virus (CAV), infectious laryngotracheitis virus (ILTV), encephalomyelitis virus or avian encephalomyelitis virus (AEV or avian leukosis virus ALV), virus of hemorragic enteritis of turkeys (HEV), pneumovirosis virus (TRTV), fowl plague virus (avian influenza), chicken hydropericarditis virus, avian reoviruses, *Escherichia coli, Mycoplasma gallinarum, Mycoplasma gallisepticum, Haemophilus avium, Pasteurella gallinarum, Pasteurella multocida gallicida,* and mixtures thereof. Preferably, for MDV the immunogen is advantageously gB and/or gD, e.g., gB and gD, for NDV the immunogen is advantageously HN and/or F, e.g., HN and F; for IBDV the immunogen advantageously isVP2; for IBV the immunogen is advantageously S (more advantageously S1) and/or M and/or N, e.g., S (or S1) and M and/or N; for CAV the immunogen is advantageously VP1 and/or VP2; for ILTV the immunogen is advantageously gB and/or gD; for AEV the immunogen advantageously is env and/or gag/pro, e.g., env and gag/pro or gag/pro; for HEV the immunogen is advantageously the 100K protein and/or hexon; for TRTV the immunogen is advantageously F and/or G, and for fowl plague the immunogen is advantageously HA and/or N and/or NP, e.g., HA and N and/or NP.

Bovine antigens, epitopes or immunogens according to the description can be derived from BHV-1, BRV, bPI-3 and/or BCV virus or a bovine pathogen selected from the group including but not limited to bovine respiratory syncytial virus and bovine viral diarrhea virus. BRSV immunogens can be BRSV F or G or N, such as BRSV F and/or G or N and/or G. BHV-1 immunogens can be gB and/or gC and/or gD. BVDV immunogens can be E0 the protein (gp48) and/or the E2 protein (gp53). The BVDV can be type 1 and/or type 2. The bPI-3 immunogens can be bPI-3 F and/or HN. See also U.S. Patents Nos. 6,451,770, 6,376,473, 6,224,878, regarding immunogens of bovine pathogens and nucleic acid molecules coding therefor and constructs that express the same.

Canine antigens, epitopes or immunogens according to the description can be derived from measles disease virus, canine distemper virus (CDV), canine parainfluenza type 2 virus (CPI-2), canine herpesvirus type I (CHV-1), rabies virus (rhabdovirus), canine parvovirus (CPV), canine coronavirus (CCV), canine adenovirus, *Borrelia burgdorferi, Leptospira* and mixtures thereof. Preferably, for CDV the immunogen is advantageously F and/or HA (*see also* U.S. Patent Nos. 6,309,647, 5,756,102 regarding CDV immunogens and constructs); for CPV the immunogen is advantageously VP2; for CCV the immunogen is advantageously S and/or M; for CHV-1 the immunogen is advantageously gB and/or gC and/or gD *(see also* U.S. Patent No. 5,688,920, 5,529,780, regarding CHV immunogens and constructs); for rabies virus the immunogen is advantageously G (*see also* U.S. Patent No. 5,843,456 regarding rabies combination compositions); for *Borrelia burgdoferi* the immunogen is advantageously OspA (*see also* U.S. Patent No. 6,368,603 regarding OspA combination compositions).

Equine antigens, epitopes or immunogens according to the description can be derived from an EHV-1 and/or EHV-4 virus or another equine pathogen selected from the group including but not limited to equine influenza virus (EIV), eastern encephalomyelitis virus (EEV), western encephalomyelitis virus (WEV), Venezuelan encephalomyelitis virus (VEV), Lyme disease agent, *Borrelia burgdorferi, Clostridium tetani, equine arteritis* virus (EAV) and rabies virus. Antigens, epitopes or immunogens can be EHV glycoproteins such as gB, gD, gB+gD, gC, and gE, for EIV the immunogen is advantageously HA, NP and/or N; for viruses of encephalitis, the immunogen is advantageously C and/or E1 and/or E2; and for *Clostridium tetani* the immunogen is all or part of the subunit C of the tetanic toxin. Reference is made to U.S. Patents Nos. 6,395,283, 6,248,333, 5,338,683, 6,183,750 for immunogens of equine pathogens and nucleic acid molecules coding therefor and consturcts that express the same.

Feline antigens, epitopes or immunogens according to the description can be derived from feline herpesvirus type 1 (FHV-1), feline calicivirus (FCV), rabies virus (rhabdovirus), feline parvovirus (FPV), feline infectious peritonitis virus (FIPV), feline leukaemia virus (FeLV), feline immunodeficiency virus (FIV), *Chlamydia* and mixtures thereof. Preferably, for FeLV the immunogen is advantageously env and/or gag and/or pol, e.g., gag/pol; for FPV the immunogen is advantageously VP2; for FIPV the immunogen is advantageously S and/or M and/or N, e.g., S and M and/or N (*see also* U.S. Patents Nos. 6,348,196 and 5,858,373 and immunogens and constructs thereof); for FHV the immunogen is advantageously gB and/or gC and/or gD, e.g., gB and gC and/or gD (*see also* U.S. Patents Nos. 5,338,683, 6,183,750; for herpesvirus immunogens and constructs expressing the same); for FCV the immunogen is advantageously C; for FIV the immunogen is advantageously env and/or gag and/or pro, e.g., gag/pro, env, or env and gag/pro (*see* also immunogens and constructs discussed in Tartaglia et al., U.S. application Serial No. 08/746,668, filed November 14, 1996); for rabies virus the immunogen is advantageously G.

Porcine antigens, epitopes or immunogens according to the description can be derived from PRRS virus and/or a porcine pathogen be selected from the group including but not limited to pseudorabies virus, porcine influenza virus, porcine parvovirus, transmissible gastro-enteritis virus (coronavirus), porcine circovirus such as porcine circovirus type 2, rotavirus, porcine adenovirus type 3, *Escherichia coli, Erysipelothrix rhusiopathiae, Bordetella bronchiseptica, Clostridium spp., Salmonella spp., Haemophilus parasuis, Pasteurella multocida, Streptococcus suis, Mycoplasma hyopneumoniae* and *Actinobacillus pleuropneumoniae.* Antigens, epitopes or immunogens of porcine pathogens can include pseudorabies virus gB, pseudorabies virus gC, pseudorabies virus gD, swine influenza HA, swine influenza NA, swine influenza NP, ORF4 of porcine reproductive and respiratory syndrome virus, ORF7 of porcine reproductive and respiratory syndrome virus, ORF5 of PRRSV, PRRSV ORF3, PRRSV ORF6, PRRSV open reading frames 5 (ORF5) and 6 (ORF6), PRRSV open reading frames 5 (ORF5) and 3 (ORF3) and 6 (ORF6), Hog Cholera Virus E1, Hog Cholera Virus E2 gene, parvovirus VP2, porcine circovirus type 2 ORF1, or porcine circovirus type 2 ORF2. Reference is made to U.S. Patents Nos. 6,517,843, 6,497,883, 6,391,314, 6,379,676, 6,217,883, 6,207,165 and U.S. Patent publication 2003003112 and WO99/53047, WO99/08706, WO01/83737, and WO00/47756 for immunogens of porcine pathogens, nucleic acid molecules coding therefor and constructs expressing the same.

The DNA plasmids of the present invention can express one or more of the West Nile Virus ("WNV") polynucleotides encoding E, prM, M or combinations or polyproteins thereof, (e.g., E, or E and prM, or E and M, or E and prM and M, or polyprotein E-prM-M, or polyprotein prM-E, or polyprotein M-E, or at least an epitope thereof. According to an embodiment of the invention, the other vector or vectors in the preparation comprises, consists essentially of or consists of a polynucleotide that encodes, and under appropriate circumstances the vector expresses one or more other proteins of the WN virus, e.g. prM, M, prM-M, or an epitope thereof.

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a protein, a polypeptide, a peptide, an epitope, a hapten, or any combination thereof. Alternately, the immunogen or antigen may comprise a toxin or antitoxin.

The term "immunogenic protein or peptide" as used herein also refers includes peptides and polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the description comprises or consists essentially of or consists of at least one epitope or antigenic determinant. The term epitope relates to a protein site able to induce an immune reaction of the humoral type (B cells) and/or cellular type (T cells).

The term "immunogenic protein or peptide" further contemplates deletions, additions and substitutions to the sequence, so long as the polypeptide functions to produce an immunological response as defined herein. In this regard, particularly preferred substitutions will generally be conservative in nature, *i.e.,* those substitutions that take place within a family of amino acids. For example, amino acids are generally divided into four families: (I) acidic-aspartate and glutamate; (2) basic--lysine, arginine, histidine; (3) non-polar-alanine, valine, leucine, isoleucine, proline, phenylalanine, methionine, tryptophan; and (4) uncharged polar--glycine, asparagine, glutamine, cystine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The terms "immunogenic" protein or polypeptide as used herein also refers to an amino acid sequence which elicits an immunological response as described above. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, *e.g.,* Epitope Mapping Protocols in Methods in Molecular Biology, Vol. 66 (Glenn E. Morris, Ed., 1996) Humana Press, Totowa, NJ. For example, linear epitopes may be determined by *e.g.,* concurrently synthesizing large numbers of peptides on solid supports, the peptides corresponding to portions of the protein molecule, and reacting the peptides with antibodies while the peptides are still attached to the supports. Such techniques are known in the art and described in, *e.g.,* U.S. Pat. No. 4,708,871; Geysen et al. (1984) Proc. Natl. Acad. Sci. USA 81:3998-4002; Geysen et al. (1986) Molec. Immunol. 23:709-715. Similarly, conformational epitopes are readily identified by determining spatial conformation of amino acids such as by, e.g., x-ray crystallography and 2-dimensional nuclear magnetic resonance. See, *e.g.,* Epitope Mapping Protocols, *supra.* Methods especially applicable to the proteins of *T. parva* are fully described in the PCT Application Serial No. PCT/US2004/022605.

Synthetic antigens are also included within the definition, for example, polyepitopes, flanking epitopes, and other recombinant antigens. See, *e.g.,* Bergmann et al. (1993) Eur. J. Immunol. 23:2777-2781; Bergmann et al. (1996) J. Immunol. 157:3242-3249; Suhrbier, A. (1997) Immunol. and Cell Biol. 75:402-408; Gardner et al. (1998) 12th World AIDS Conference, Geneva, Switzerland, Jun. 28-Jul. 3, 1998. Immunogenic fragments, for purposes of the present description, will usually include at least about 3 amino acids, preferably at least about 5 amino acids, more preferably at least about 10-15 amino acids, and most preferably 25 or more amino acids, of the molecule. There is no critical upper limit to the length of the fragment, which could comprise nearly the full-length of the protein sequence, or even a fusion protein comprising at least one epitope of the protein.

Accordingly, a minimum structure of a polynucleotide expressing an epitope is that it comprises or consists essentially of or consists of nucleotides to encode an epitope or antigenic determinant of the protein or polyprotein of interest. A polynucleotide encoding a fragment of the total protein or polyprotein, more advantageously, comprises or consists essentially of or consists of a minimum of 21 nucleotides, advantageously at least 42 nucleotides, and preferably at least 57, 87 or 150 consecutive or contiguous nucleotides of the sequence encoding the total protein or polyprotein. Epitope determination procedures, such as, generating overlapping peptide libraries (Hemmer B. et al., Immunology Today, 1998, 19 (4), 163-168), Pepscan (Geysen et al., (1984) Proc. Nat. Acad. Sci. USA, 81, 3998-4002; Geysen et al., (1985) Proc. Nat. Acad. Sci. USA, 82, 178-182; Van der Zee R. et al., (1989) Eur. J. Immunol., 19, 43-47; Geysen H.M., (1990) Southeast Asian J. Trop. Med. Public Health, 21, 523-533; Multipin.RTM. Peptide Synthesis Kits de Chiron) and algorithms (De Groot A. et al., (1999) Nature Biotechnology, 17, 533-561), and in PCT Application Serial No. PCT/U52004/022605 can be used in the practice of the description, without undue experimentation. Other documents cited herein may also be consulted for methods for determining epitopes of an immunogen or antigen and thus nucleic acid molecules that encode such epitopes.

A "polynucleotide" is a polymeric form of nucleotides of any length, which contain deoxyribonucleotides, ribonucleotides, and analogs in any combination. Polynucleotides may have three-dimensional structure, and may perform any function, known or unknown. The term "polynucleotide" includes double-, single-stranded, and triple-helical molecules. Unless otherwise specified or required, any embodiment of the description described herein that is a polynucleotide encompasses both the double stranded form and each of two complementary forms known or predicted to make up the double stranded form of either the DNA, RNA or hybrid-molecule.

The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A polynucleotide may comprise modified nucleotides, such as methylated nucleotides and nucleotide analogs, uracyl, other sugars and linking groups such as fluororibose and thiolate, and nucleotide branches. The sequence of nucleotides may be further modified after polymerization, such as by conjugation, with a labeling component. Other types of modifications included in this definition are caps, substitution of one or more of the naturally occurring nucleotides with an analog, and introduction of means for attaching the polynucleotide to proteins, metal ions, labeling components, other polynucleotides or solid support. The polynucleotides can be obtained by chemical synthesis or derived from a microorganism.

The description further comprises a complementary strand to a polynucleotide encoding an antigen, epitope, immunogen, peptide or polypeptide of interest. The complementary strand can be polymeric and of any length, and can contain deoxyribonucleotides, ribonucleotides, and analogs in any combination.

The terms "protein"; "peptide", "polypeptide" and "polypeptide fragment" are used interchangeably herein to refer to polymers of amino acid residues of any length. The polymer can be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

An "isolated" polynucleotide or polypeptide is one that is substantially free of the materials with which it is associated in its native environment. By substantially free, is meant at least 50%, advantageously at least 70%, more advantageously at least 80%, and even more advantageously at least 90% free of these materials.

Hybridization reactions can be performed under conditions of different "stringency." Conditions that increase stringency of a hybridization reaction are well known. See for example, "Molecular Cloning: A Laboratory Manual", second edition (Sambrook et al. 1989). Examples of relevant conditions include (in order of increasing stringency): incubation temperatures of 25°C, 37°C, 50°C, and 68°C; buffer concentrations of 10 x SSC, 6 x SSC, 1 x SSC, 0.1 x SSC (where SSC is 0.1 5 M NaCl and 15 mM citrate buffer) and their equivalent using other buffer systems; formamide concentrations of 0%, 25%, 50%, and 75%; incubation times from 5 minutes to 24 hours; 1, 2 or more washing steps; wash incubation times of 1, 2, or 15 minutes; and wash solutions of 6 x SSC, 1 x SSC, 0.1 x SSC, or deionized water.

The description further encompasses polynucleotides encoding functionally equivalent variants and derivatives of the polypeptides of interest and functionally equivalent fragments thereof which may enhance, decrease or not significantly affect properties of the polypeptides encoded thereby. These functionally equivalent variants, derivatives, and fragments display the ability to retain biological activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serine/threonine/methionine; lysine/arginine; and phenylalanine/tyrosine/tryptophan. In one embodiment, the variants have at least 50%, at least 55%, at least 60%, at least 65%, at least 70%, at least 75%, at least 80%, at least 85%, at least 86%, at least 87%, at least 88%, at least 89%, at least 90%, at least 91 %, at least 92%, at least 93%, at least 94%, at least 95%, at least 96%, at least 97%, at least 98% or at least 99% homology or identity to the polynucleotide or polypeptide of interest.

For the purposes of the present description, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990; 87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson & Lipman, Proc. Natl. Acad. Sci. USA 1988; 85: 2444-2448.

Advantageous for use according to the present description is the WU-BLAST (Washington University BLAST) version 2.0 software. WU-BLAST version 2.0 executable programs for several UNIX platforms can be downloaded from ftp ://blast.wustl.edu/blast/executables. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990; 215: 403-410; Gish & States, 1993;Nature Genetics 3: 266-272; Karlin & Altschul, 1993;Proc. Natl. Acad. Sci. USA 90: 5873-5877).

In general, comparison of amino acid sequences is accomplished by aligning an amino acid sequence of a polypeptide of a known structure with the amino acid sequence of a the polypeptide of unknown structure. Amino acids in the sequences are then compared and groups of amino acids that are homologous are grouped together. This method detects conserved regions of the polypeptides and accounts for amino acid insertions and deletions. Homology between amino acid sequences can be determined by using commercially available algorithms (see also the description of homology above). In addition to those otherwise mentioned herein, mention is made too of the programs BLAST, gapped BLAST, BLASTN, BLASTP, and PSI-BLAST, provided by the National Center for Biotechnology Information. These programs are widely used in the art for this purpose and can align homologous regions of two amino acid sequences.

In all search programs in the suite the gapped alignment routines are integral to the database search itself. Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology " or "identity", for instance, with respect to a nucleotide or amino acid sequence, can indicate a quantitative measure of homology between two sequences. The percent sequence homology can be calculated as (*N_{ref} - N_{dif}*)*100/N*_{ref}*, wherein N*_{dif}* is the total number of non-identical residues in the two sequences when aligned and wherein N*_{ref}* is the number of residues in one of the sequences. Hence, the DNA sequence AGTCAGTC will have a sequence identity of 75% with the sequence AATCAATC (N*_{ref}* = 8; N*_{dif}=*2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur & Lipman, Proc Natl Acad Sci USA 1983; 80:726), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the description and can be derived from DNA sequences, by thymidine (T) in the DNA sequence being considered equal to uracil (U) in RNA sequences.

And, without undue experimentation, the skilled artisan can consult with many other programs or references for determining percent homology.

The description further encompasses the polynucleotides of interest contained in a vector molecule or an expression vector and operably linked to a promoter element and optionally to an enhancer.

A "vector" refers to a recombinant DNA or RNA plasmid or virus that comprises a heterologous polynucleotide to be delivered to a target cell, either in vitro or in vivo. The heterologous polynucleotide may comprise a sequence of interest for purposes of therapy, and may optionally be in the form of an expression cassette. As used herein, a vector needs not be capable of replication in the ultimate target cell or subject. The term includes cloning vectors also included are viral vectors.

The term "recombinant" means a polynucleotides semisynthetic, or synthetic origin which either does not occur in nature or is linked to another polynucleotide in an arrangement not found in nature.

"Heterologous" means derived from a genetically distinct entity from the rest of the entity to which it is being compared. For example, a polynucleotide, may be placed by genetic engineering techniques into a plasmid or vector derived from a different source, and is a heterologous polynucleotide. A promoter removed from its native coding sequence and operatively linked to a coding sequence other than the native sequence is a heterologous promoter.

The polynucleotides of the description may comprise additional sequences, such as additional encoding sequences within the same transcription unit, controlling elements such as promoters, ribosome binding sites, polyadenylation sites, additional transcription units under control of the same or a different promoter, sequences that permit cloning, expression, homologous recombination, and transformation of a host cell, and any such construct as may be desirable to provide embodiments of this description.

Elements for the expression of an antigen, epitope, immunogen, peptide or polypeptide of interest are advantageously present in a vector. In minimum manner, this comprises, consists essentially of, or consists of an initiation codon (ATG), a stop codon and a promoter, and optionally also a polyadenylation sequence for certain vectors such as plasmid and certain viral vectors, e.g., viral vectors other than poxviruses. When the polynucleotide encodes a polyprotein fragment, e.g. a peptide, advantageously, in the vector, an ATG is placed at 5' of the reading frame and a stop codon is placed at 3'. Other elements for controlling expression may be present, such as enhancer sequences, stabilizing sequences, such as intron and signal sequences permitting the secretion of the protein.

Methods for making and/or administering a vector or recombinants or plasmid for expression of gene products of genes of the description either in *vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450 and 6; 312,683; U.S. patent application Serial No. 920,197, filed October 16,1986; WO 90/01543; W091/11525; WO 94/16716; WO 96/39491; WO 98/33510; EP 265785; EP 0 370 573; Andreansky et al., Proc. Natl. Acad. Sci. USA 1996;93:11313-11318; Ballay et al., EMBO J. 1993;4:3861-65; Felgner et al., J. Biol. Chem. 1994;269:2550-2561; Frolov et al., Proc. Natl. Acad. Sci. USA 1996;93:11371-11377; Graham, Tibtech 1990;8:85-87; Grunhaus et al., Sem. Virol. 1992;3:237-52; Ju et al., Diabetologia 1998;41:736-739; Kitson et al., J. Virol. 1991;65:3068-3075; McClements et al., Proc. Natl. Acad. Sci. USA 1996;93:11414-11420; Moss, Proc. Natl. Acad. Sci. USA 1996;93:11341-11348; Paoletti, Proc. Natl. Acad. Sci. USA 1996;93:11349-11353; Pennock et al., Mol. Cell. Biol. 1984;4:399-406; Richardson (Ed), Methods in Molecular Biology 1995;39, "Baculovirus Expression Protocols," HumanaPress Inc.; Smith et al. (1983) Mol. Cell. Biol. 1983;3:2156-2165; Robertson et al., Proc. Natl. Acad. Sci. USA 1996;93:11334-11340; Robinson et al., Sem. Immunol. 1997;9:271; and Roizman, Proc. Natl. Acad. Sci. USA 1996;93:11307-11312. The herein cited documents, in addition to providing examples of vectors useful in the practice of the description, can also provide sources for peptides or fragments thereof to be expressed by vector or vectors in, or included in, the compositions of the description.

The present description also relates to preparations comprising vectors, such as expression vectors, e.g., therapeutic compositions. The preparations can comprise, consist essentially of, or consist of one or more vectors, e.g., expression vectors, such as *in vivo* expression vectors, comprising, consisting essentially or consisting of (and advantageously expressing) one or more of antigens, epitopes or immunogens of interest. Advantageously, the vector contains and expresses a polynucleotide that includes, consists essentially of, or consists of a polynucleotide coding for (and advantageously expressing) an antigen, epitope, immunogen, peptide or polypeptide of interest, in a pharmaceutically or veterinarily acceptable carrier, excipient or vehicle. Thus, according to an embodiment of the description, the other vector or vectors in the preparation comprises, consists essentially of or consists of a polynucleotide that encodes, and under appropriate circumstances the vector expresses one or more other proteins of an antigen, epitope, immunogen, peptide or polypeptide of interest or a fragment thereof.

According to another embodiment, the vector or vectors in the preparation comprise, or consist essentially of, or consist of polynucleotide(s) encoding one or more proteins or fragment(s) thereof of an antigen, epitope, immunogen, peptide or polypeptide of interest the vector or vectors expressing the polynucleotide(s). The preparation advantageously comprises, consists essentially of, or consists of, at least two vectors comprising, consisting essentially of, or consisting of, and advantageously also expressing, advantageously *in vivo* under appropriate conditions or suitable conditions or in a suitable host cell, polynucleotides from different isolates encoding the same proteins and/or for different proteins, but advantageously the same proteins. Preparations containing one or more vectors containing, consisting essentially of or consisting of polynucleotides encoding, and advantageously expressing, advantageously *in vivo,* an antigen or fusion protein of interest or an epitope thereof. The description is also directed at mixtures of vectors that contain, consist essentially of, or consist of coding for, and express, different antigens, epitopes or immunogens, e.g., an antigen, epitope, immunogen, peptide or polypeptide from different species such as, but not limited to, birds, dogs, cats, cows, horses, humans and pigs.

According to another embodiment of the description, the expression vectors are expression vectors used for the *in vitro* expression of proteins in an appropriate cell system. The expressed proteins can be harvested in or from the culture supernatant after, or not after secretion (if there is no secretion a cell lysis typically occurs or is performed), optionally concentrated by concentration methods such as ultrafiltration and/or purified by purification means, such as affinity, ion exchange or gel filtration-type chromatography methods.

A "host cell" denotes a prokaryotic or eukaryotic cell that has been genetically altered, or is capable of being genetically altered by administration of an exogenous polynucleotide, such as a recombinant plasmid or vector. When referring to genetically altered cells, the term refers both to the originally altered cell and to the progeny thereof. Advantageous host cells include, but are not limited to, baby hamster kidney (BHK) cells, colon carcinoma (Caco-2) cells, COS7 cells, MCF-7 cells, MCF-10A cells, Madin-Darby canine kidney (MDCK) cells, Madin-Darby bovine kidnet cells (MDBK), mink lung (Mv1Lu) cells, MRC-5 cells, U937 cells and VERO cells, Chinese Hamster Ovary (CHO) KI cells. Polynucleotides comprising a desired sequence can be inserted into a suitable cloning or expression vector, and the vector in turn can be introduced into a suitable host cell for replication and amplification. Polynucleotides can be introduced into host cells by any means known in the art. The vectors containing the polynucleotides of interest can be introduced into the host cell by any of a number of appropriate means, including direct uptake, endocytosis, transfection, nucleofection, f-mating, electroporation, transfection employing calcium chloride, rubidium chloride, calcium phosphate, DEAE-dextran, or other substances; microprojectile bombardment; lipofection; and infection (where the vector is infectious, for instance, a retroviral vector). The choice of introducing vectors or polynucleotides will often depend on features of the host cell.

In an advantageous embodiment, the description provides for the administration of a therapeutically effective amount of a formulation for the delivery and expression of an antigen, epitope, immunogen, peptide or polypeptide of interest in a target cell. Determination of the therapeutically effective amount is routine experimentation for one of ordinary skill in the art. In one embodiment, the formulation comprises an expression vector comprising a polynucleotide that expresses an antigen, epitope, immunogen, peptide or polypeptide of interest and a pharmaceutically or veterinarily acceptable carrier, vehicle or excipient In an advantageous embodiment, the pharmaceutically or veterinarily acceptable carrier, vehicle or excipient facilitates transfection and/or improves preservation of the vector or protein.

The pharmaceutically or veterinarily acceptable carriers or vehicles or excipients are well known to the one skilled in the art. For example, a pharmaceutically or veterinarily acceptable carrier or vehicle or excipient can be a 0.9% NaCl (e.g., saline) solution or a phosphate buffer. Other pharmaceutically or veterinarily acceptable carrier or vehicle or excipients that can be used for methods of this description include, but are not limited to, poly-(L-glutamate) or polyvinylpyrrolidone. The pharmaceutically or veterinarily acceptable carrier or vehicle or excipients may be any compound or combination of compounds facilitating the administration of the vector (or protein expressed from a vector *in vitro*); advantageously, the carrier, vehicle or excipient may facilitate transfection and/or improve preservation of the vector (or protein). Doses and dose volumes are herein discussed in the general description and can also be determined by the skilled artisan from this disclosure read in conjunction with the knowledge in the art, without any undue experimentation.

The cationic lipids containing a quaternary ammonium salt which are advantageously but not exclusively suitable for plasmids, are advantageously those having the following formula: 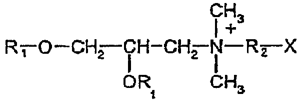 in which R₁ is a saturated or unsaturated straight-chain aliphatic radical having 12 to 18 carbon atoms, R₂ is another aliphatic radical containing 2 or 3 carbon atoms and X is an amine or hydroxyl group, e.g. the DMRIE. In another embodiment the cationic lipid can be associated with a neutral lipid, e.g. the DOPE.

Among these cationic lipids, preference is given to DMRIE (N-(2-hydroxyethyl)-N,N-dimethyl-2,3-bis(tetradecyloxy)-1-propane ammonium; WO96/34109), advantageously associated with a neutral lipid, advantageously DOPE (dioleoyl-phosphatidyl-ethanol amine; Behr J. P., 1994, Bioconjugate Chemistry, 5, 382-389), to form DMRIE-DOPE.

Advantageously, the plasmid mixture with the adjuvant is formed extemporaneously and advantageously contemporaneously with administration of the preparation or shortly before administration of the preparation; for instance, shortly before or prior to administration, the plasmid-adjuvant mixture is formed, advantageously so as to give enough time prior to administration for the mixture to form a complex, e.g. between about 10 and about 60 minutes prior to administration, such as approximately 30 minutes prior to administration.

When DOPE is present, the DMRIE:DOPE molar ratio is advantageously about 95: about 5 to about 5:about 95, more advantageously about 1: about 1, e.g., 1:1.

The DMRIE or DMRIE-DOPE adjuvant:plasmid weight ratio can be between about 50: about 1 and about 1: about 10, such as about 10: about 1 and about 1:about 5, and advantageously about 1: about 1 and about 1: about 2, e.g., 1:1 and 1:2.

The immunogenic compositions and vaccines according to the description may comprise or consist essentially of one or more adjuvants. Suitable adjuvants for use in the practice of the present description are (1) polymers of acrylic or methacrylic acid, maleic anhydride and alkenyl derivative polymers, (2) immunostimulating sequences (ISS), such as oligodeoxyribonucleotide sequences having one ore more non-methylated CpG units (Klinman et al., Proc. Natl. Acad. Sci., USA, 1996, 93, 2879-2883; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminium phosphate, (7) saponin or (8) other adjuvants discussed in any document cited in the instant application, or (9) any combinations or mixtures thereof.

The oil in water emulsion (3), which is especially appropriate for viral vectors, can be based on: light liquid paraffin oil (European pharmacopoeia type), isoprenoid oil such as squalane, squalene, oil resulting from the oligomerization of alkenes, e.g. isobutene or decene, esters of acids or alcohols having a straight-chain alkyl group, such as vegetable oils, ethyl oleate, propylene glycol, di(caprylate/caprate), glycerol tri(caprylate/caprate) and propylene glycol dioleate, or esters of branched, fatty alcohols or acids, especially isostearic acid esters.

The oil is used in combination with emulsifiers to form an emulsion. The emulsifiers may be nonionic surfactants, such as: esters of on the one hand sorbitan, mannide (e.g. anhydromannitol oleate), glycerol, polyglycerol or propylene glycol and on the other hand oleic, isostearic, ricinoleic or hydroxystearic acids, said esters being optionally ethoxylated, or polyoxypropylene-polyoxyethylene copolymer blocks, such as Pluronic, e.g., L121.

Among the type (1) adjuvant polymers, preference is given to polymers of crosslinked acrylic or methacrylic acid, especially crosslinked by polyalkenyl ethers of sugars or polyalcohols. These compounds are known under the name carbomer (Phanneuropa, vol. 8, no. 2, June 1996). One skilled in the art can also refer to U.S. Patent No. 2,909,462, which provides such acrylic polymers crosslinked by a polyhydroxyl compound having at least three hydroxyl groups, preferably no more than eight such groups, the hydrogen atoms of at least three hydroxyl groups being replaced by unsaturated, aliphatic radicals having at least two carbon atoms. The preferred radicals are those containing 2 to 4 carbon atoms, e.g. vinyls, allyls and other ethylenically unsaturated groups. The unsaturated radicals can also contain other substituents, such as methyl. Products sold under the name Carbopol (BF Goodrich, Ohio, USA) are especially suitable. They are crosslinked by allyl saccharose or by allyl pentaerythritol. Among them, reference is made to Carbopol 974P, 934P and 971P.

As to the maleic anhydride-alkenyl derivative copolymers, preference is given to EMA (Monsanto), which are straight-chain or crosslinked ethylene-maleic anhydride copolymers and they are, for example, crosslinked by divinyl ether. Reference is also made to J. Fields et al., Nature 186: 778-780, June 4, 1960.

With regard to structure, the acrylic or methacrylic acid polymers and EMA are preferably formed by basic units having the following formula: 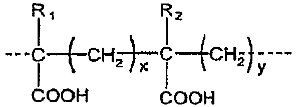 in which:
- R₁ and R₂, which can be the same or different, represent H or CH₃
- x = 0 or 1, preferably x = 1
- y=1 or 2, with x + y = 2.

For EMA, x = 0 and y = 2 and for carbomers x = y = 1.

These polymers are soluble in water or physiological salt solution (20 g/l NaCl) and the pH can be adjusted to 7.3 to 7.4, e.g., by soda (NaOH), to provide the adjuvant solution in which the expression vector(s) can be incorporated. The polymer concentration in the final vaccine composition can range between 0.01 and 1.5% w/v, advantageously 0.05 to 1% w/v and preferably 0.1 to 0.4% w/v.

The cytokine or cytokines (5) can be in protein form in the immunogenic or vaccine composition, or can be co-expressed in the host with the immunogen or immunogens or epitope(s) thereof. Preference is given to the co-expression of the cytokine or cytokines, either by the same vector as that expressing the immunogen or immunogens or epitope(s) thereof, or by a separate vector therefor.

The description comprehends preparing such combination compositions; for instance by admixing the active components, advantageously together and with an adjuvant, carrier, cytokine, and/or diluent.

Cytokines that may be used in the present description include, but are not limited to, granulocyte colony stimulating factor (G-CSF), granulocyte/macrophage colony stimulating factor (GM-CSF), interferon α (IFN α), interferon β (IFN β), interferon γ, (IFN γ), interleukin-1α (IL-1 α), interleukin-1 β (IL-1β), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), tumor necrosis factor α (TNF α), tumor necrosis factor β (TNF β), and transforming growth factor β (TGF β). It is understood that cytokines can be co-administered and/or sequentially administered with the immunogenic or vaccine composition of the present description. Thus, for instance, the vaccine of the instant description can also contain an exogenous nucleic acid molecule that expresses in vivo a suitable cytokine, e.g., a cytokine matched to this host to be vaccinated or in which an immunological response is to be elicited (for instance, a canine cytokine for preparations to be administered to dogs).

Advantageously, the pharmaceutical and/or therapeutic compositions and/or formulations according to the description comprise or consist essentially of or consist of an effective quantity to elicit a therapeutic response of one or more expression vectors and/or polypeptides as discussed herein; and, an effective quantity can be determined from this disclosure, including the documents cited herein, and the knowledge in the art, without undue experimentation.

In the case of therapeutic and/or pharmaceutical compositions based on a plasmid vector, a dose can comprise, consist essentially of or consist of, in general terms, about in 1 µg to about 2000 µg, advantageously about 50 µg to about 1000 µg and more advantageously from about 100 µg to about 800 µg of plasmid expressing the antigen, epitope, immunogen, peptide or polypeptide of interest When the therapeutic and/or pharmaceutical compositions based on a plasmid vector is administered with electroporation the dose of plasmid is generally between about 0.1 µg and 1mg, advantageously between about 1 µg and 100 µg, advantageously between about 2 µg and 50 µg. The dose volumes can be between about 0.1 and about 2 ml, advantageously between about 0.2 and about 1 ml. These doses and dose volumes are suitable for the treatment mammalian target species.

In an advantageous embodiment, an animal is vaccinated with two doses of inactivated vaccine at about 3 to 4 week intervals via the subcutaneous route, although an intramuscular route is also contemplated. Blood samples may be collected on the day of the first and/or second vaccination and about 2 to 4 weeks after the second vaccination to determine the levels of specific antibodies by methods known to one of skill in the art, for example, virus neutralization, hemagglutination inhibition, ELISA or single radial heamolysis (SRH) tests.

It should be understood by one of skill in the art that the disclosure herein is provided by way of example and the present description is not limited thereto. From the disclosure herein and the knowledge in the art, the skilled artisan can determine the number of administrations, the administration route, and the doses to be used for each injection protocol, without any undue experimentation.

The present description contemplates at least one administration to an animal of an efficient amount of the therapeutic composition made according to the description. The animal may be male, female, pregnant female and newborn. This administration may be via various routes including, but not limited to, intramuscular (IM), intradermal (ID) or subcutaneous (SC) injection or via intranasal or oral administration. The therapeutic composition according to the description can also be administered by a needleless apparatus (as, for example with a Pigjet, Biojector or Vitajet apparatus (Bioject, Oregon, USA)). Another approach to administer plasmid compositions is to use electroporation (see, e.g. S. Tollefsen et al. Vaccine, 2002, 20, 3370-3378; S. Tollefsen et al. Scand. J. Immunol., 2003, 57, 229-238; S. Babiuk et al., Vaccine, 2002, 20, 3399-3408; PCT Application No. WO99/01158). In another embodiment, the therapeutic composition is delivered to the animal by gene gun or gold particle bombardment. In an advantageous embodiment, the animal is a vertebrate.

One embodiment of the description is a method of eliciting an immune response against the antigen, epitope, immunogen, peptide or polypeptide of interest in an animal, comprising administering formulation for delivery and expression of a recombinant vaccine in an effective amount for eliciting an immune response. Still another embodiment of the description is a method of inducing an immunological or protective response in an animal, comprising administering to the animal an effective amount of a formulation for delivery and expression of an antigen, epitope, immunogen, peptide or polypeptide of interest wherein the formulation comprises a recombinant vaccine and a pharmaceutically or veterinarily acceptable carrier, vehicle or excipient.

The description relates to a method to elicit, induce or stimulate the immune response of an animal, advantageously a mammal or a vertebrate.

Another embodiment of the description is a kit for performing a method of inducing an immunological or protective response against antigen, epitope, immunogen, peptide or polypeptide of interest in an animal comprising a recombinant vaccine and instructions for performing the method of delivery in an effective amount for eliciting an immune response in the animal.

The invention will now be further described by way of the following non-limiting examples.

### EXAMPLE 1: Impact Of Kanamycin Resistance ("Kanar") Gene Expression Efficiency On Plasmid Replication Efficiency In Bacteria

The following example illustrates the impact ofKanamycin resistance ("KanaR") gene expression efficiency on plasmid replication efficiency in bacteria.

First, the KanaR expression cassette elements were analyzed, specifically promoter(s), translation initiation and transcription termination. Upon analysis of these expression elements, these elements are modified and the impact of the modified elements on plasmid production yields are analyzed. Four potential promoters were identified. P3 (SEQ ID NO: 3) was identified from database and literature searches, the structure being described in GenBank AN# X00928 (SEQ ID NO: 4) but not detected by current softwares. P2 (SEQ ID NO: 2) was determined by bioinformatic analysis and was also found in the published GenBank AN# V00359 (SEQ ID NO: 5). P1 (SEQ ID NO: 1) was an additional potential promoter belonging to the pVR1012 backbone. P0 was another potential promoter belonging to the pVR1012 backbone. FIG. 1 shows how P2 (SEQ ID NO: 2) and P3 partially overlap.

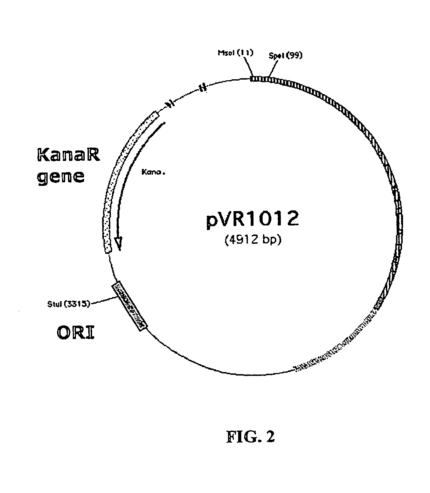

There is no transcription termination signal sequence for KanaR in pVR1012. This could have many potential consequences. Many KanaR transcripts could be abnormally long and degraded as there would be a useless metabolic burden. Moreover, KanaR expression could be sub-optimal in pVR1012. KanaR transcription may continue through the replication origin, ORI, and interfere with replication initiation as shown schematically in FIG. 2. Therefore, two appropriate transcription terminators were identified: rrnBT1+T2, which can be found in GenBank AN# U13872 (SEQ ID NO: 6) and is used in many commercial expression vectors, and speA, which has Acc. Number M31770 (SEQ ID NO: 7). Furthermore, the ATG initiator codon of pVR1012 was replaced by TTG in order to lower translation initiation efficacy. Thus, the aim was to determine the effect of each modified expression cassette on the kanamycin resistance capacity and plasmid production yields.

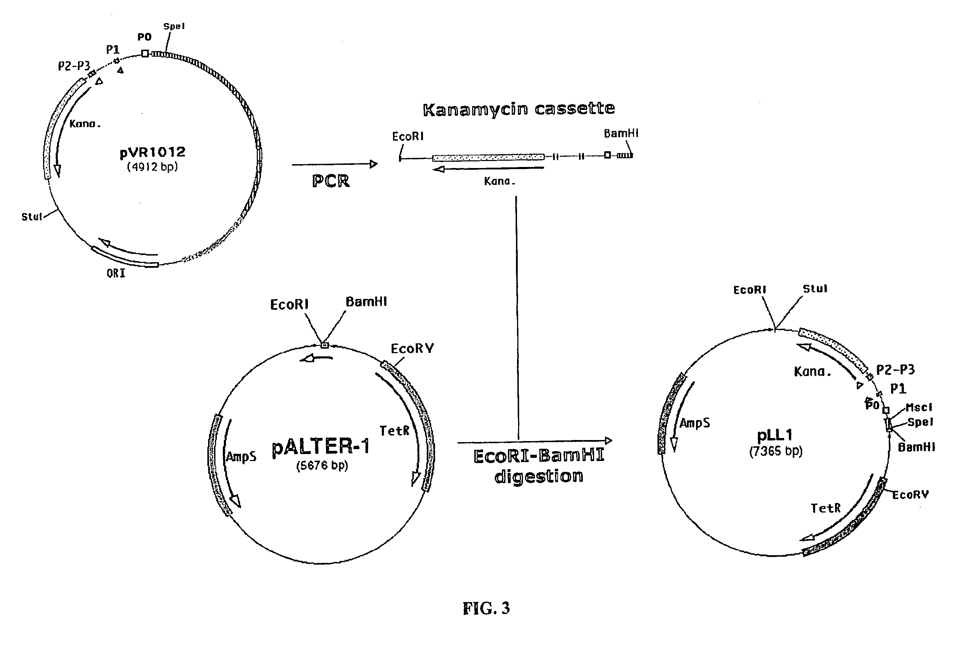

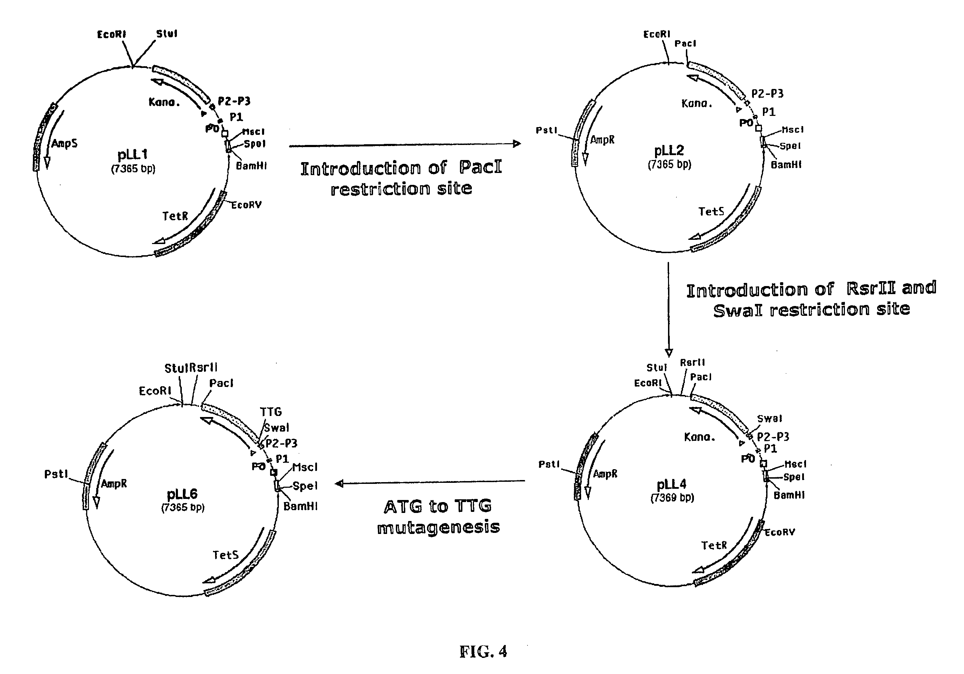

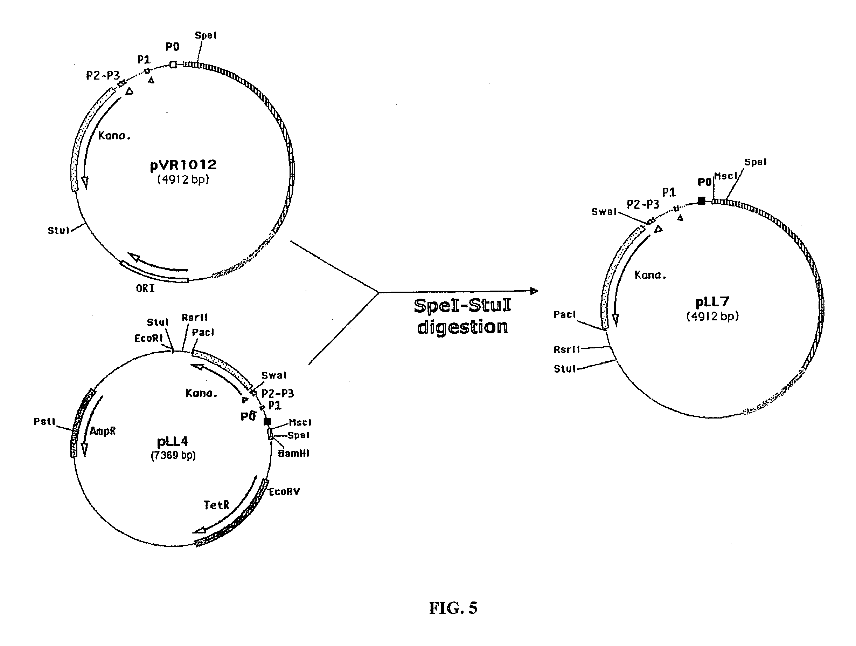

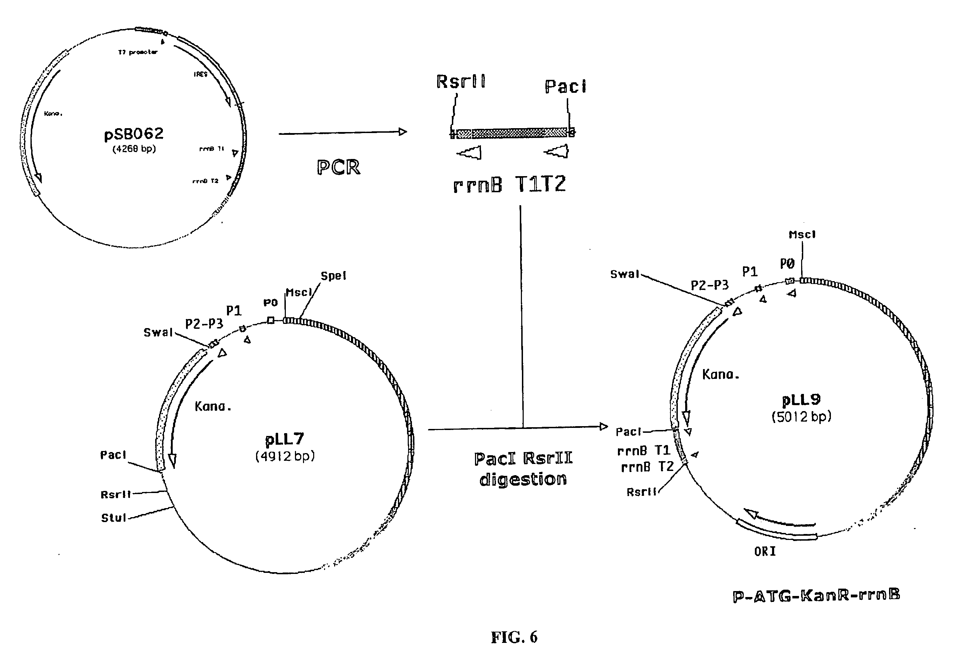

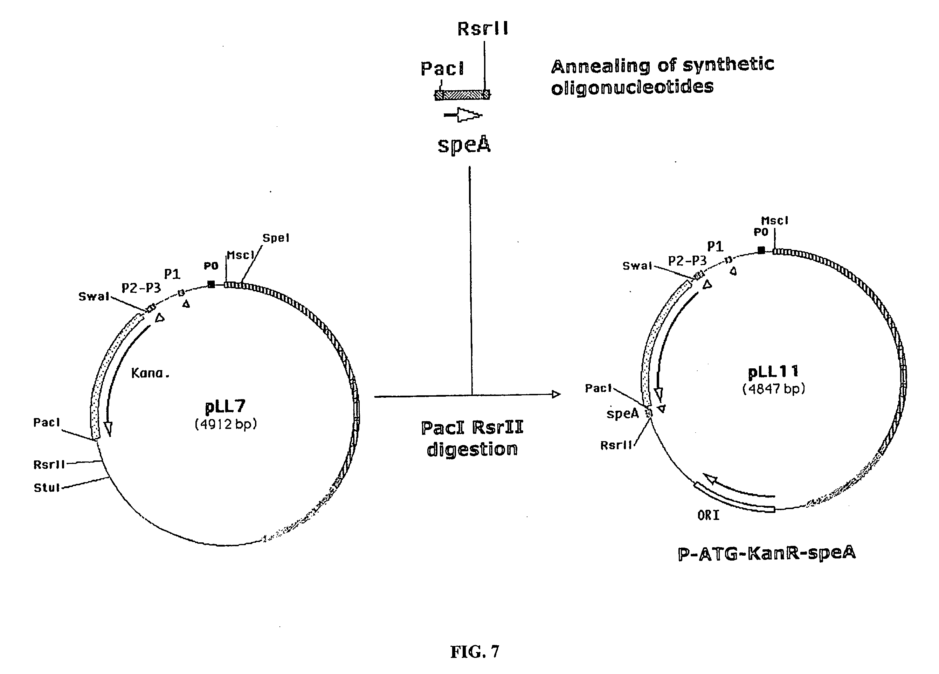

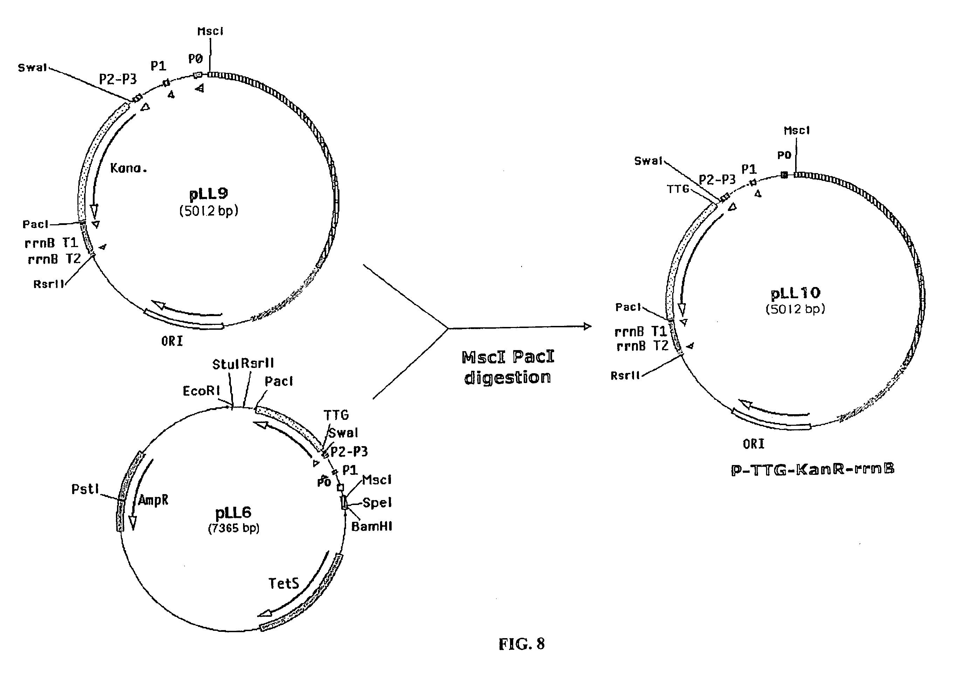

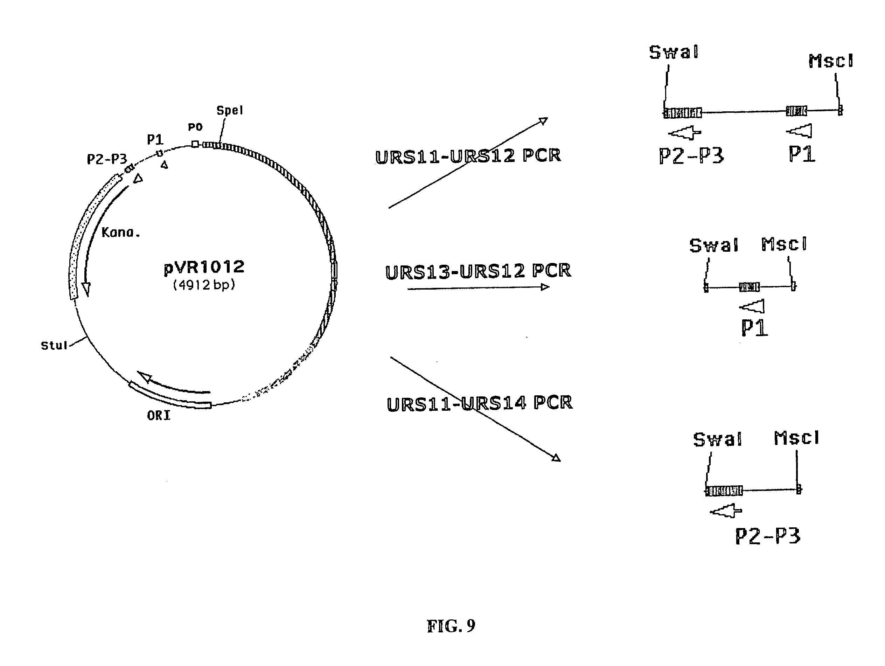

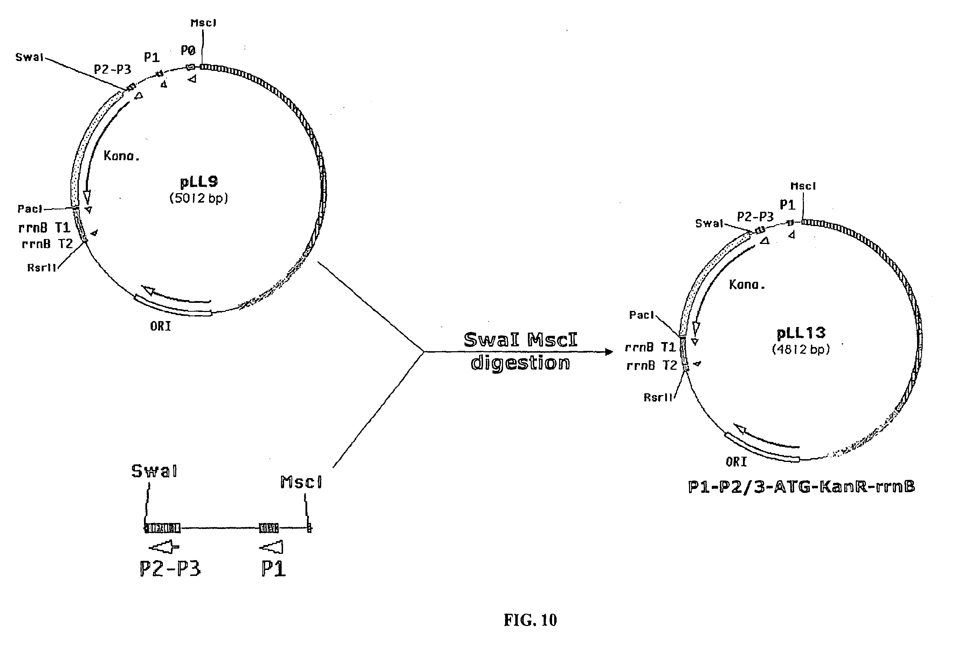

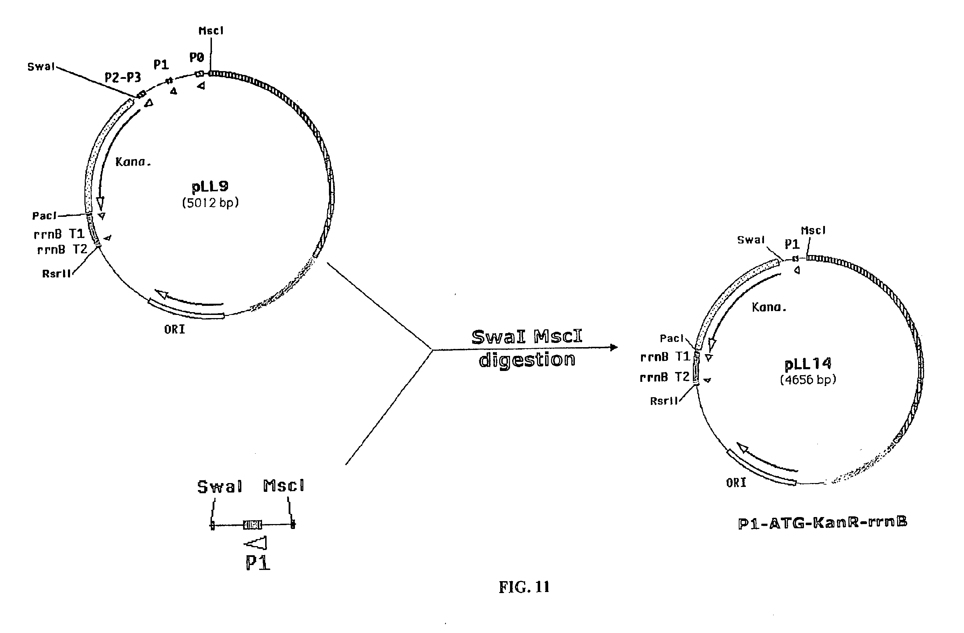

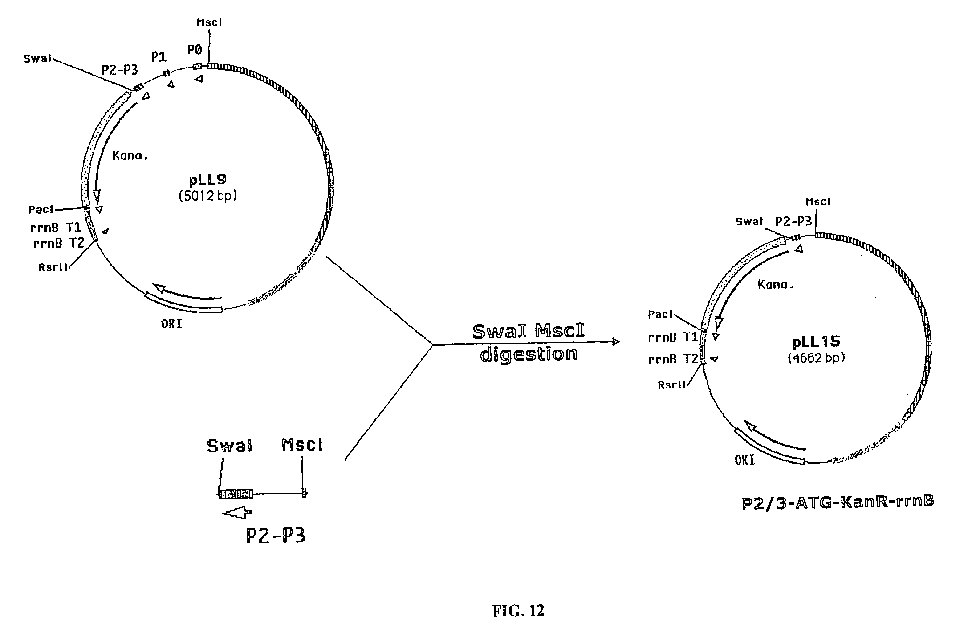

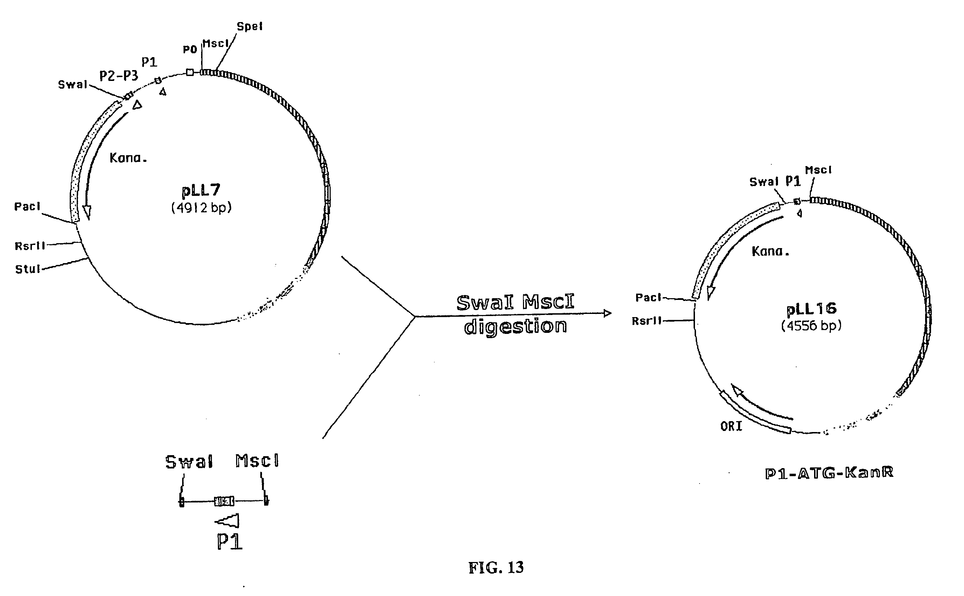

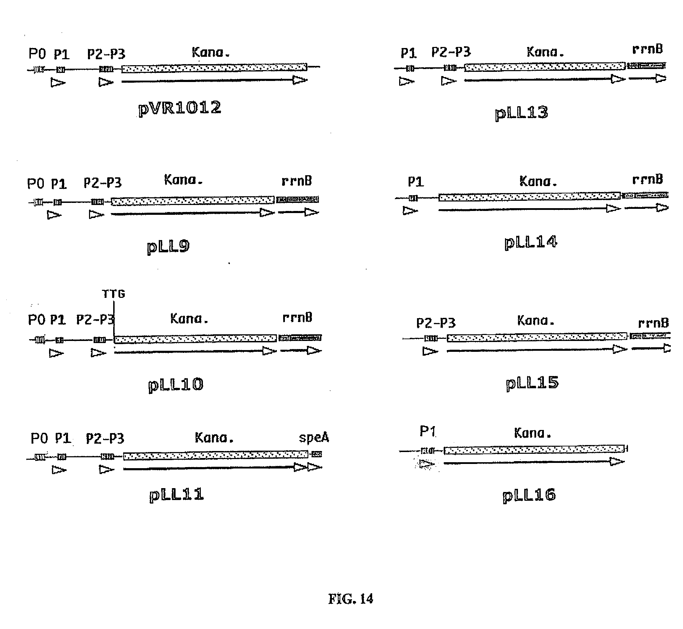

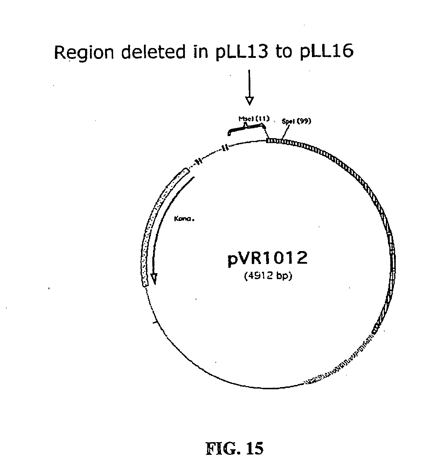

Thus, PVR1012 derivatives were generated with promoter P1 (SEQ ID NO: 1), P2/P3 (SEQ ID NOS: 2-3), or P1 (SEQ ID NO: 1) with P2/P3 (SEQ ID NOS: 2-3), transcription terminators rrnBT1+T2 or speA, and initiator codon TTG. In order to introduce such DNA sequences upstream or downstream the KanaR ORF, single restriction sites were needed. Such sites were not found in pVR1012 so they were introduced by site-directed mutagenesis. First, the KanaR cassette was cloned into the mutagenesis vector pALTER-1, as shown in FIG. 3. FIG. 4 shows how a *PacI* restriction site was introduced downstream of the KanaR ORF thereby producing pLL2, a *SwaI* restriction site was introduced upstream and a *RsrII* restriction site was introduced downstream thereby producing pLL4, and finally the translation initiator codon ATG was mutated to TTG, producing pLL6. The mutated KanaR cassette of pLL4 was then cloned into pVR1012 to form pLL7, as shown in FIG. 5. The rrnB terminator sequence was then cloned into pLL7 by PCR from pSB062 and subsequent *PacI RsrII* digestion to form pLL9, as shown in FIG. 6. FIG. 7 shows the cloning of the speA terminator sequence into pLL7 by annealing of synthetic oligonucleotides and *PacI RsrII* digestion to form pLL11. Next, the mutated translation initiation sequence of pLL6 was cloned into pLL9 by *MscI PacI* digestion to form pLL10, shown in FIG. 8. Finally, FIG. 9 illustrates how promoter sequences P2/P3 (SEQ ID NOS: 2-3) with P1 (SEQ ID NO: 1), P1 (SEQ ID NO: 1), and P2/P3 (SEQ ID NOS: 2-3) were cloned by URS11-URS12 PCR, URS13-URS12 PCR and URS11-URS14 PCR, respectively. FIGS. 10, 11 and 12 show how these were then incorporated into the pLL9 to form pLL13, 14 and 15, respectively. Similarly, FIG. 13 shows how P1 (SEQ ID NO: 1) was incorporated into pLL7 to form pLL16. FIG. 14 shows several of the resulting constructs. The pLL 13, pLL14, pLL15 and pLL16 constructs had a 192 base pair deletion between the CMV promoter and the Kana P1 (SEQ ID NO: 1) promoter as illustrated by FIG. 15. Table 1 summarizes these constructs..

**Table 1**

| | **KanaR gene** | | | **Insert** | **Plasmid size (bp)** |
|---|---|---|---|---|---|
| **PLASMID** | **Promoter(s)** | **Initiator codon** | **Terminator** | | |
| pVR1012 | P0, P1, P2/3 | ATG | no | no | 4911 |
| pLL7 | P0, P1, P2/3 | ATG | no | no | 4911 |
| pLL9 | P0, P1, P2/3 | ATG | ***rrnB T1 - T2*** | no | 5011 |
| pLL10 | P0, P1, P2/3 | TTG | ***rrnB T1 - T2*** | no | 5011 |
| pLL11 | P0, P1, P2/3 | ATG | ***speA*** | no | 4846 |
| pLL13* | **P1, P2**/3 | ATG | ***rrnB* T1 - T2** | no | 4811 |
| pLL14* | **P1** | ATG | ***rrnB* T1 - T2** | no | 4655 |
| pLL15* | **P2/3** | ATG | ***rrnB* T1 - T2** | no | 4661 |
| pLL16* | **P1** | ATG | no | no | 4555 |
| pPB662 | P0, P1, P2/3 | ATG | no | FIV env | 7456 |
| pLL19* | **P1** | ATG | ***rrnB* T1 - T2** | FIV env | 7204 |

| | | | | | |
|---|---|---|---|---|---|
| * Constructs having a 192 bp deletion between the CMV promoter and the Kana P1 promoter | | | | | |

The effect of the modifications on the KanaR gene transcript size was confirmed by Northern blot experiments. These confirmed the heterogeneity of pVR1012 KanaR transcripts as shown by the smear. They also confirmed the presence of three active promoters in pLL9, pLL10 and pLL11: one major short transcript from P2/P3 (SEQ ID NOS: 2-3) and two weaker transcripts from P0 and P1 (SEQ ID NO: 1). As expected, pLL13 generated two transcripts and pLL14 and pLL15 only one.

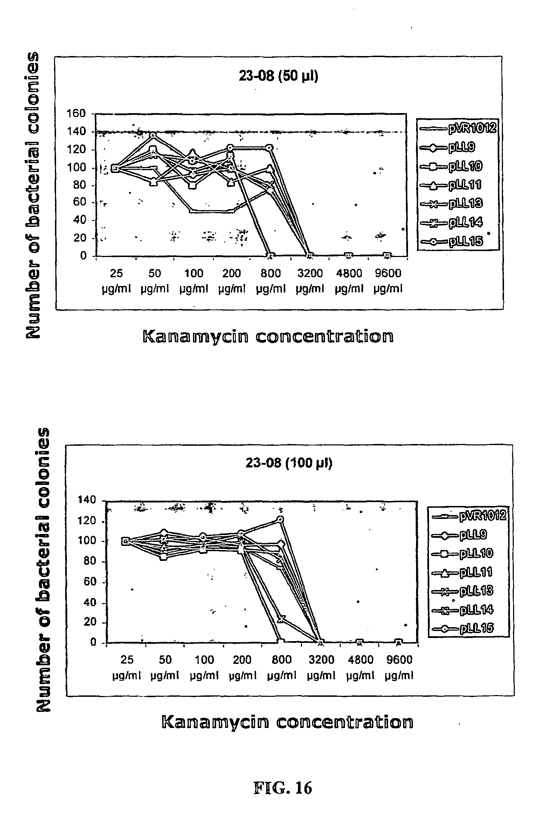

Several parameters were then tested on the pVR1012 derivatives in order to assess their new characteristics. The ability to resist increasing Kanamycin concentrations was tested by first inoculating one bacterial colony containing each construct, pVR1012 to pLL15, in LB medium and growing overnight at 37°C and 200 RPM. Bacteria in each suspension were quantified by spectrophotometry (OD₆₀₀ nm). Appropriate dilutions of bacterial suspensions, 50 or 100 µl, were plated on LB medium containing increasing Kanamycin concentrations. Finally, colonies were counted, the results of which are shown in FIG. 16. These results showed that bacteria containing most pVR1012 derivatives had the same capacity to resist increasing Kana concentration without adaptation as growth was not affected between 0 and 800 µg/ml and was inhibited at 3200 µg/ml. Bacteria containing pLL10 and pLL14 had a significantly lowered capacity to resist Kanamycin as growth was 90% to 100% inhibited at 800 µg/ml, but not at 200 µg/ml. Thus, it was concluded that the absence of P2/P3 (SEQ ID NOS: 2-3) promoters or lowered translation efficiencies has a significant impact on the KanaR enzyme production, indicating that P2/P3 (SEQ ID NOS: 2-3) promoters are most efficient for KanaR expression and the TTG inhibitor lowers KanaR expression. However, these mutations still enable bacterial resistance and growth in the presence of classical Kana concentrations.

EGLI medium adaptation capacity was tested by first growing bacterial cell suspensions containing each bacterial construct and 10⁴ CFU in LB, and thereafter plating on EGLI plates. 25 to 50 isolated colonies containing each construct were subsequently plated on a new EGLI plate. The growing colonies were counted, the results of which are shown in Table 2. The pLL10, pLL14 and pLL16 plasmid backbones confer a clear growth advantage in nutrient-limited medium to bacteria containing them. Futhermore, lower Kanamycin production capacity is associated to better adaptation capacity on low nutrient medium in the presence of Kana 100 µg/ml.

**Table 2**

| | | Growth on EGLI plates | | Growth in EGLI broth | | Plasmid integrity | | |
|---|---|---|---|---|---|---|---|---|
| | Construct | Nb. of colonies grown/ Nb of colonies plated | percentage growth | Nb. Of clones grown/ Nb of clones inoculated | percentage growth | Number of mutant detected | Percentage of mutant detected | Mutant reference |
| Experiment 1 | pVR1012-1 | 20/30 | 67 | 15/15 | 100 | 15/15 | 100 | all |
| | pLL9 | 22/30 | 73 | 15/15 | 100 | 3/13 | 23 | pLL9#3, #4 and #15 |
| | pLL10 | 40/40 | **100** | 15/15 | 100 | 0/15 | **0** | - |
| | pLL11 | 36/40 | 90 | 11/15 | 73 | 1/11 | 9 | pLL11#3 |
| | pLL13 | 34/40 | 85 | 8/15 | 53 | 3/8 | 38 | pLL13#1, #3 and #11 |
| | pLL14 | 30/30 | 100 | 15/15 | 100 | 0/15 | **0** | - |
| | pLL15 | 45/50 | 90 | 13/15 | 87 | 2/13 | 15 | pLL15#3 and #14 |
| Experiment 2 | pVR1012-11 | 11/25 | 44 | 6/11 | 55 | 1/6 | 17 | pVR1012#1 0 |
| | pLL16 | 25/25 | **100** | 25/25 | 100 | 0/25 | 0 | - |
| Experiment 3 | pVR1012-III | 25/25 | **100** | 25/25 | 100 | 9/25 | 36 | pVR1012#2, #4, #8, #9, #11, #13, #14, #18 and #24 |
| | pPB662 | 25/25 | **100** | 25/25 | 100 | 0/25 | **0** | - |
| | pLL14 | 25/25 | 100 | 25/25 | 100 | 0/25 | **0** | - |
| | pLL19 | 25/25 | **100** | 20/25 | 80 | 0/20 | **0** | - |

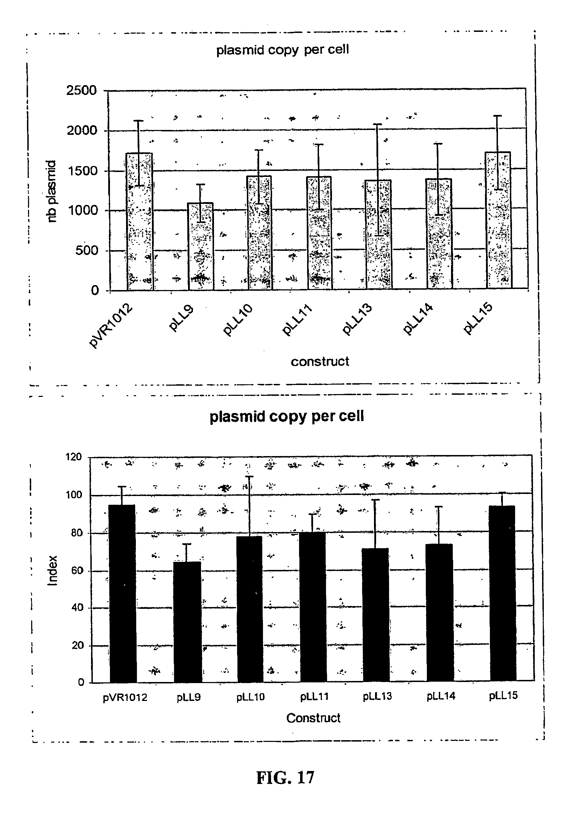

Plasmid yields obtained under laboratory growth conditions were tested by first growing one colony of bacteria containing each construct in liquid LB medium with Kana 100 µg/ml overnight at 37°C and 200 RPM. The amount of bacteria in each suspension was evaluated by spectrophotometry, OD₆₀₀. DNA from each suspension was purified using Eppendorf columns and quantified by spectrophotometry, OD₂₆₀, each DNA solution being quantified twice. The number of plasmid copies in each suspension was then calculated, the results of which can be seen in FIG. 17. No significant differences were observed between the different constructs after growth in the LB medium with Kana 100 µg/ml. Plasmid stability was further evaluated by submitting three plasmid preparations derived from each construct to restriction analysis.

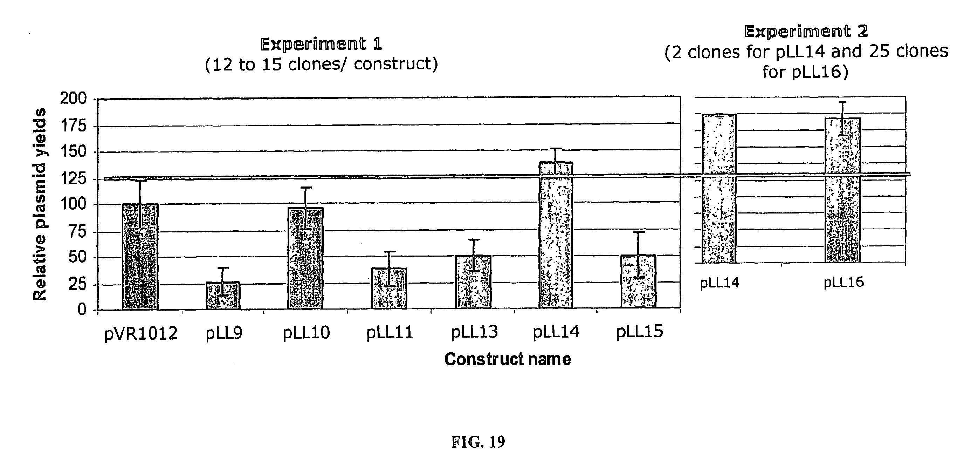

Plasmid yields obtained under laboratory growth conditions were tested by first adapting EGLI on solid medium. 15 and 25 colonies, Experiment A and B respectively, containing each construct were grown overnight in liquid EGLI medium at 37°C and 200 RPM. The amount of bacteria in each suspension was evaluated by spectrophotometry, OD₆₀₀. DNA from each suspension was purified using Qiagen columns and quantified by spectrophotometry, OD₂₆₀. The number of plasmid copies in each suspension was then deduced, the results of which can be seen in FIG. 19.

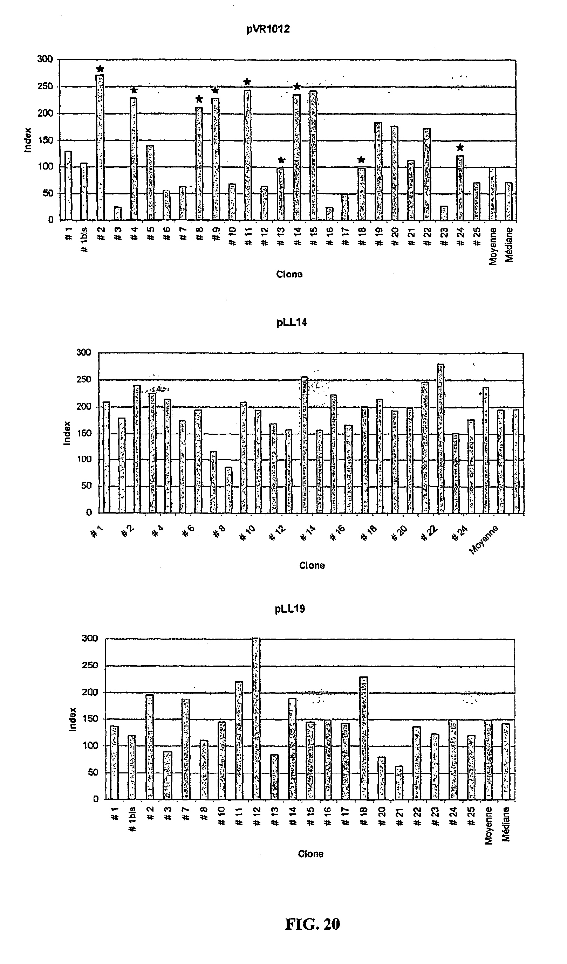

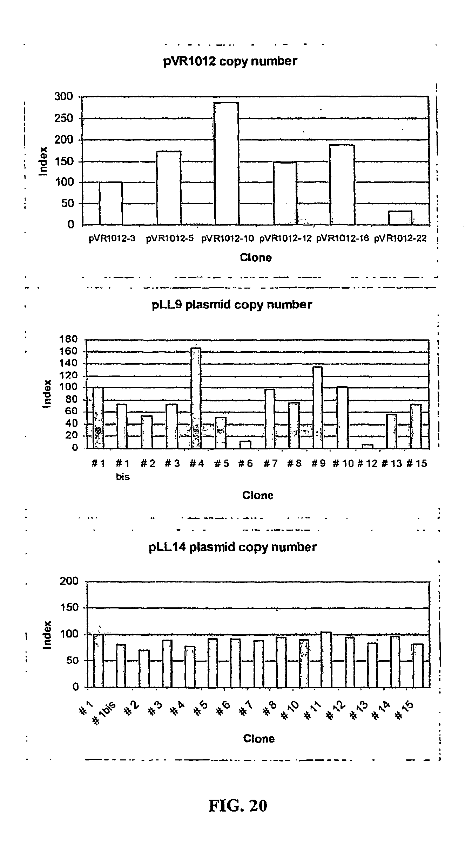

The results show that constructs pLL14 and pLL16 are the best candiates with respect to plasmid yields under laboratory conditions in EGLI medium. Furthermore, plasmid yields were significantly lower when constructs contained a transcription terminator and efficient translation initiation (ATG). Concomitantly, plasmid yields were highest when constructs did not contain a translation terminator or did contain the suboptimal P1 (SEQ ID NO: 1) promoter or suboptimal translation initiation (TTG). Plasmid yields were comparable in constructs pVR1012 and pLL10, indicating that low translation efficiency compensated the negative effect of the transcription terminator. These results suggest that the lower the capacity to express KanaR, the higher the plasmid yields are. In an extension of the test, plasmid stability was further evaluated by submitting each plasmid preparations derived from each construct to restriction analysis. Table 2 shows the results of this analysis. No mutation was detected after EGLI adaptation for pLL10, pLL14, pLL16 and pLL19 whereas it was more than 9% for the other constructs. It was also found that there was increased homogeneity of plasmid yields in pLL14, pLL16 and pLL19 EGLI-adapted clones. FIG. 20 illustrates the difference between the high heterogeneity of plasmid yield with constructs pVR1012 and a lower heterogeneity with construct pLL14 or pLL19.

Plasmid yields obtained by fermentation were tested by first arbitrarily selecting one clone of EGLI-adapted SCS1/pLL14 for fermentation. After fermentation, the bacteria were centrifuged and plasmid was extracted from an aliquot and quantified twice according to the methods developed by PGEA. The results showed that the growth rate of SCS1/pLL14 was significantly lower than that usually observed for pVR1012 derivatives, such as pPB266 and pPB662. Furthermore, the specific plasmid yield of pLL14 was of 3.1 mg plasmid/g of bacterial pellet. This result corresponds to the best results ever obtained with pVR1012 derivatives after Kana adaptation and clone selection, such as pPB266. Plasmid stability was then evaluated by submitting the plasmid preparations from each fermented clone to restriction analysis with three different restriction digestion patterns. Table 4 provides a summary of the overall plasmid analysis results. The results show that the level of KanaR expression had an impact on plasmid replication efficiency. The lower the expression, the higher replication rates were in nutrient-low medium and Kana 100 µg/ml. Lower KanaR expression could be obtained by deleting the natural promoter (P2/P3 (SEQ ID NOS: 2-3)), ensuring the absence of a transcription termination signal and mutating the translation initiation. Low KanaR expression led to up to 50% increased plasmid yields as compared to the parental plasmid backbone pVR1012. It also led to increased plasmid stability and homogeneity in plasmid production yields. This therefore probably allowed for limited clone selection.

**Table 4**

| **Plasmid name** | **Promoter** | **Transcrip. terminator** | **Transl. Init.** | **Analysis** | | | |
|---|---|---|---|---|---|---|---|
| | | | | **Growth on increasing [Kana]: MIC** | **Plasmid yields ("Erlen" growth in Egli/ Kana 100)** | **Mutation rates** | **Plasmid yields (fermentor)** |
| pVR1012 | P0, P1 + P2/3 | NO | ATG | 1600 to 3200 | 48** and 51*** | 17 to 100 | ND |
| pLL9 | P0, P1 +P2/3 | rrnB T1-T2 | ATG | 1600 to 3200 | 19 | 23 | |
| pLL10 | P0, P1 + P2/3 | rrnB T1-T2 | TTG | 400 to 800 | 67 | 0 | |
| pLL11 | P0, P1 + P2/3 | SpeA | ATG | 1600 to 3200 | 26 | 9 | |
| pLL13* | P1 + P2/3 | rrnB T1-T2 | ATG | 1600 to 3200 | 35 | 38 | |
| pLL14* | P1 | rrnB T1-T2 | ATG | 400 to 800 | 100 | 0 | 3.1 mg/g of bacterial pellet |
| pLL15* | P2/3 | rrnB T1-T2 | ATG | 1600 to 3200 | 35 | 15 | ND |
| pLL16* | P1 | NO | ATG | | 98** | 0 | 1.9 mg/g of bacterial pellet |
| PLL19* | P1 | RrnB T1-T2 | ATG | - | 76*** | 0 | 2.9 mg/g of bacterial pellet |

| | | | | | | | |
|---|---|---|---|---|---|---|---|
| * Constructs having a 192 bp deletion between the CMV promoter and the Kana P1 promoter. ** Results obtained in a second plasmid yield quantification test. *** Results obtained in a third plasmid yield quantification test. | | | | | | | |

Plasmid pLL16 is subject to further analysis, such as resistance to increasing Kana concentrations and plasmid yields after fermentation. Construction of pLL17 and pLL18 is also completed, wherein pLL17 has the P1 promoter, a TTG initiation codon and terminator rrnB T1/2 and pLL18 has the P1 promoter, a TTG initiation codon and no terminator.

However, these constructs may be difficult to obtain due to low KanaR capacity. These constructions are analyzed using the previously described tests. Plasmid yields tests after Kana adaption on selected constructs (e.g., pLL14 to -18) are performed. RNA transcript analysis from the KanaR gene of all constructions is performed to confirm the deduced model. By flipping the KanaR cassette within the pVR1012, it can be confirmed that the KanaR cassette has no impact on the replication origin.

### EXAMPLE 2: Sequences

DEFINITION Transposon Tn2350 Km(r) gene 5' region from plasmid R1.
ACCESSION X00928 (SEQ ID NO: 4) 
1 gaattcccgc agattaacgc gcataacaag cggtttactc gttttggcct gcaatgtaac
61 ataatataca ttatgcgcac taaggtagag gcagcaagat tatgcggttt tgatcagaac
121 tcagttaacc atcccgggat tctgctctgg cccattcagc gcagtttttt actttggatg
181 aagttaaccc atgttatatt gcacaagata aaaatatatc atcatgaaca ataaaactgt
 
241 ctgcttacat aaacagtaat acaaggggtg ttatgagcca tattcaacgg gaaacgtctt
301 gctcgaggga attc
DEFINITION Transposon Tn903.
ACCESSION V00359 J01839 (SEQ ID NO: 5) 
1 ggctttgttg aataaatcag atttcgggta agtctccccc gtagcgggtt gtgttttcag
61 gcaatacgca cgctttcagg catacctgct ttcgtcattt tgttcagcgc tcgtaccagg
121 gccatagcct ccgcaacctg accatcgtag tcacgcagcg tcagtgaacc cccgaacagc
181 tgttttaccc ggtacatcgc cgtttccgct atcgagcgac ggttgtaatc tgttgtccat
241 ttccaccgcg cattactccc ggtcattcgc tgattagcca ctgcacggtt acggtctgca
301 tattcaccgg gccagtaacc cgcacctttt cggggaggga taagcgcgct gattttctta
361 cgccgcagtt catcgtgaca gagccgggtg tcgtaagcgc cgtctgccga tgctgccctg
421 atttttctgt gagtctgccg gataagaccc gggaaggctt ctgagtcggt cacattgttc
481 agcgacaggt cagcgcagat gatttcatgt gttttactgt caacggcgag atgcagctta
541 cgccagatac ggcggcgttc ctggccatgc tttttgactt tccattcgcc ttcaccaaag
601 accttcagcc cggtggaatc aatcaccaga tgcgcgattt caccccgggt gaacgttttg
661 aaactgatat taaccgactt tgcccgcctg ctgacacagc tgtaatccgg gcagcgcaac
721 ggaacattca tcagtgtaaa aatggaatca ataaagccct gcgcagcgcg cagggtcagc
781 ctgaatacgc gtttaatgac cagcacagtc gtgatggcaa ggtcagaata gcgctgaggt
841 ctgcctcgtg aagaaggtgt tgctgactca taccaggcct gaatcgcccc atcatccagc
901 cagaaagtga gggagccacg gttgatgaga gctttgttgt aggtggacca gttggtgatt
961 ttgaactttt gctttgccac ggaacggtct gcgttgtcgg gaagatgcgt gatctgatcc
1021 ttcaactcag caaaagttcg atttattcaa caaagccacg ttgtgtctca aaatctctga
1081 tgttacattg cacaagataa aaatatatca tcatgaacaa taaaactgtc tgcttacata
1141 aacagtaata caaggggtgt tatgagccat attcaacggg aaacgtcttg ctcgaggccg
1201 cgattaaatt ccaacatgga tgctgattta tatgggtata aatgggctcg cgataatgtc
1261 gggcaatcag gtgcgacaat ctatcgattg tatgggaagc ccgatgcgcc agagttgttt
1321 ctgaaacatg gcaaaggtag cgttgccaat gatgttacag atgagatggt cagactaaac
1381 tggctgacgg aatttatgcc tcttccgacc atcaagcatt ttatccgtac tcctgatgat
1441 gcatggttac tcaccactgc gatccccggg aaaacagcat tccaggtatt agaagaatat
1501 cctgattcag gtgaaaatat tgttgatgcg ctggcagtgt tcctgcgccg gttgcattcg
1561 attcctgttt gtaattgtcc ttttaacagc gatcgcgtat ttcgtctcgc tcaggcgcaa
1621 tcacgaatga ataacggttt ggttgatgcg agtgattttg atgacgagcg taatggctgg
1681 cctgttgaac aagtctggaa agaaatgcat aagcttttgc cattctcacc ggattcagtc
1741 gtcactcatg gtgatttctc acttgataac cttatttttg acgaggggaa attaataggt
1801 tgtattgatg ttggacgagt cggaatcgca gaccgatacc aggatcttgc catcctatgg
1861 aactgcctcg gtgagttttc tccttcatta cagaaacggc tttttcaaaa atatggtatt
1921 gataatcctg atatgaataa attgcagttt catttgatgc tcgatgagtt tttctaatca
1981 gaattggtta attggttgta acactggcag agcattacgc tgacttgacg ggacggcggc
2041 tttgttgaat aaatcgaact tttgctgagt tgaaggatca gatcacgcat cttcccgaca
2101 acgcagaccg ttccgtggca aagcaaaagt tcaaaatcac caactggtcc acctacaaca
2161 aagctctcat caaccgtggc tccctcactt tctggctgga tgatggggcg attcaggcct
2221 ggtatgagtc agcaacacct tcttcacgag gcagacctca gcgctattct gaccttgcca
2281 tcacgactgt gctggtcatt aaacgcgtat tcaggctgac cctgcgcgct gcgcagggct
2341 ttattgattc catttttaca ctgatgaatg ttccgttgcg ctgcccggat tacagctgtg
2401 tcagcaggcg ggcaaagtcg gttaatatca gtttcaaaac gttcacccgg ggtgaaatcg
2461 cgcatctggt gattgattcc accgggctga aggtctttgg tgaaggcgaa tggaaagtca
2521 aaaagcatgg ccaggaacgc cgccgtatct ggcgtaagct gcatctcgcc gttgacagta
2581 aaacacatga aatcatctgc gctgacctgt cgctgaacaa tgtgaccgac tcagaagcct
2641 tcccgggtct tatccggcag actcacagaa aaatcagggc agcatcggca gacggcgctt
2701 acgacacccg gctctgtcac gatgaactgc ggcgtaagaa aatcagcgcg cttatccctc
2761 cccgaaaagg tgcgggttac tggcccggtg aatatgcaga ccgtaaccgt gcagtggcta
2821 atcagcgaat gaccgggagt aatgcgcggt ggaaatggac aacagattac aaccgtcgct
2881 cgatagcgga aacggcgatg taccgggtaa aacagctgtt cgggggttca ctgacgctgc
2941 gtgactacga tggtcaggtt gcggaggcta tggccctggt acgagcgctg aacaaaatga
3001 cgaaagcagg tatgcctgaa agcgtgcgta ttgcctgaaa acacaacccg ctacggggga
3061 gacttacccg aaatctgatt tattcaacaa agcc
DEFINITION pTrc99a cloning vector, complete sequence.
ACCESSION U13872 (SEQ ID NO: 6) 
1 gtttgacagc ttatcatcga ctgcacggtg caccaatgct tctggcgtca ggcagccatc
61 ggaagctgtg gtatggctgt gcaggtcgta aatcactgca taattcgtgt cgctcaaggc
121 gcactcccgt tctggataat gttttttgcg ccgacatcat aacggttctg gcaaatattc
181 tgaaatgagc tgttgacaat taatcatccg gctcgtataa tgtgtggaat tgtgagcgga
241 taacaatttc acacaggaaa cagaccatgg aattcgagct cggtacccgg ggatcctcta
301 gagtcgacct gcaggcatgc aagcttggct gttttggcgg atgagagaag attttcagcc
361 tgatacagat taaatcagaa cgcagaagcg gtctgataaa acagaatttg cctggcggca
421 gtagcgcggt ggtcccacct gaccccatgc cgaactcaga agtgaaacgc cgtagcgccg
481 atggtagtgt ggggtctccc catgcgagag tagggaactg ccaggcatca aataaaacga
541 aaggctcagt cgaaagactg ggcctttcgt tttatctgtt gtttgtcggt gaacgctctc
601 ctgagtagga caaatccgcc gggagcggat ttgaacgttg cgaagcaacg gcccggaggg
661 tggcgggcag gacgcccgcc ataaactgcc aggcatcaaa ttaagcagaa ggccatcctg
721 acggatggcc tttttgcgtt tctacaaact ctttttgttt atttttctaa atacattcaa
781 atatgtatcc gctcatgaga caataaccct gataaatgct tcaataatat tgaaaaagga
841 agagtatgag tattcaacat ttccgtgtcg cccttattcc cttttttgcg gcattttgcc
901 ttcctgtttt tgctcaccca gaaacgctgg tgaaagtaaa agatgctgaa gatcagttgg
961 gtgcacgagt gggttacatc gaactggatc tcaacagcgg taagatcctt gagagttttc
1021 gccccgaaga acgttttcca atgatgagca cttttaaagt tctgctatgt ggcgcggtat
1081 tatcccgtgt tgacgccggg caagagcaac tcggtcgccg catacactat tctcagaatg
1141 acttggttga gtactcacca gtcacagaaa agcatcttac ggatggcatg acagtaagag
1201 aattatgcag tgctgccata accatgagtg ataacactgc ggccaactta cttctgacaa
1261 cgatcggagg accgaaggag ctaaccgctt ttttgcacaa catgggggat catgtaactc
1321 gccttgatcg ttgggaaccg gagctgaatg aagccatacc aaacgacgag cgtgacacca
1381 cgatgcctac agcaatggca acaacgttgc gcaaactatt aactggcgaa ctacttactc
1441 tagcttcccg gcaacaatta atagactgga tggaggcgga taaagttgca ggaccacttc
1501 tgcgctcggc ccttccggct ggctggttta ttgctgataa atctggagcc ggtgagcgtg
1561 ggtctcgcgg tatcattgca gcactggggc cagatggtaa gccctcccgt atcgtagtta
1621 tctacacgac ggggagtcag gcaactatgg atgaacgaaa tagacagatc gctgagatag
1681 gtgcctcact gattaagcat tggtaactgt cagaccaagt ttactcatat atactttaga
1741 ttgatttaaa acttcatttt taatttaaaa ggatctaggt gaagatcctt tttgataatc
1801 tcatgaccaa aatcccttaa cgtgagtttt cgttccactg agcgtcagac cccgtagaaa
1861 agatcaaagg atcttcttga gatccttttt ttctgcgcgt aatctgctgc ttgcaaacaa
1921 aaaaaccacc gctaccagcg gtggtttgtt tgccggatca agagctacca actctttttc
1981 cgaaggtaac tggcttcagc agagcgcaga taccaaatac tgtccttcta gtgtagccgt
2041 agttaggcca ccacttcaag aactctgtag caccgcctac atacctcgct ctgctaatcc
2101 tgttaccagt ggctgctgcc agtggcgata agtcgtgtct taccgggttg gactcaagac
2161 gatagttacc ggataaggcg cagcggtcgg gctgaacggg gggttcgtgc acacagccca
2221 gcttggagcg aacgacctac accgaactga gatacctaca gcgtgagcta tgagaaagcg
2281 ccacgcttcc cgaagggaga aaggcggaca ggtatccggt aagcggcagg gtcggaacag
2341 gagagcgcac gagggagctt ccagggggaa acgcctggta tctttatagt cctgtcgggt
2401 ttcgccacct ctgacttgag cgtcgatttt tgtgatgctc gtcagggggg cggagcctat
2461 ggaaaaacgc cagcaacgcg gcctttttac ggttcctggc cttttgctgg ccttttgctc
2521 acatgttctt tcctgcgtta tcccctgatt ctgtggataa ccgtattacc gcctttgagt
2581 gagctgatac cgctcgccgc agccgaacga ccgagcgcag cgagtcagtg agcgaggaag
2641 cggaagagcg cctgatgcgg tattttctcc ttacgcatct gtgcggtatt tcacaccgca
2701 tatggtgcac tctcagtaca atctgctctg atgccgcata gttaagccag tatacactcc
2761 gctatcgcta cgtgactggg tcatggctgc gccccgacac ccgccaacac ccgctgacgc
2821 gccctgacgg gcttgtctgc tcccggcatc cgcttacaga caagctgtga ccgtctccgg
2881 gagctgcatg tgtcagaggt tttcaccgtc atcaccgaaa cgcgcgaggc agcagatcaa
2941 ttcgcgcgcg aaggcgaagc ggcatgcatt tacgttgaca ccatcgaatg gtgcaaaacc
3001 tttcgcggta tggcatgata gcgcccggaa gagagtcaat tcagggtggt gaatgtgaaa
3061 ccagtaacgt tatacgatgt cgcagagtat gccggtgtct cttatcagac cgtttcccgc
3121 gtggtgaacc aggccagcca cgtttctgcg aaaacgcggg aaaaagtgga agcggcgatg
3181 gcggagctga attacattcc caaccgcgtg gcacaacaac tggcgggcaa acagtcgttg
3241 ctgattggcg ttgccacctc cagtctggcc ctgcacgcgc cgtcgcaaat tgtcgcggcg
3301 attaaatctc gcgccgatca actgggtgcc agcgtggtgg tgtcgatggt agaacgaagc
3361 ggcgtcgaag cctgtaaagc ggcggtgcac aatcttctcg cgcaacgcgt cagtgggctg
3421 atcattaact atccgctgga tgaccaggat gccattgctg tggaagctgc ctgcactaat
3481 gttccggcgt tatttcttga tgtctctgac cagacaccca tcaacagtat tattttctcc
 
3541 catgaagacg gtacgcgact gggcgtggag catctggtcg cattgggtca ccagcaaatc
3601 gcgctgttag cgggcccatt aagttctgtc tcggcgcgtc tgcgtctggc tggctggcat
3661 aaatatctca ctcgcaatca aattcagccg atagcggaac gggaaggcga ctggagtgcc
3721 atgtccggtt ttcaacaaac catgcaaatg ctgaatgagg gcatcgttcc cactgcgatg
3781 ctggttgcca acgatcagat ggcgctgggc gcaatgcgcg ccattaccga gtccgggctg
3841 cgcgttggtg cggatatctc ggtagtggga tacgacgata ccgaagacag ctcatgttat
3901 atcccgccgt taaccaccat caaacaggat tttcgcctgc tggggcaaac cagcgtggac
3961 cgcttgctgc aactctctca gggccaggcg gtgaagggca atcagctgtt gcccgtctca
4021 ctggtgaaaa gaaaaaccac cctggcgccc aatacgcaaa ccgcctctcc ccgcgcgttg
4081 gccgattcat taatgcagct ggcacgacag gtttcccgac tggaaagcgg gcagtgagcg
4141 caacgcaatt aatgtgagtt agcgcgaatt gatctg
DEFINITION E.coli arginine decarboxylase (speA) gene, complete cds, agmatinase (speB) and methionine adenosyltransferase (metK) genes, 5' end.
ACCESSION M31770 (SEQ ID NO: 7)

1 tacccaaggt cgctggtggt gatttcgccg ccaactaaaa ccaatgccgg tttttacgta
61 ggtttcgcaa gcaacgcgtg ctttcggatc ctgttcgagg atcgcgtcta aaacggcatc
121 agaaatttgg tcagcaattt tgtcaggatg cccttcagag acggactcgg acgtaaaaag
181 gtgttttgcc atatttaata tcacctaaag agaatttggt tagctcaaac tgttgtgtgg
241 attttctgtg gtagcggatc ctaccacgac tctgcaggtt aaaaacactg gcagtctgag
301 tgttaatcgg tatggatgga ttaacatctg gatggctatt ttaggtcaat tcttcaccct
361 atttccactt ttttttgaat cgtgtctcat tctgttaaaa acgtggctgg aaatttttcc
421 tgacaatgcc ggcattctgc gtatttatct tttgcaattt tctgccattg tggggtataa
481 aacgcggcgc gcggcttaaa taaaaagcac acgacgtttc tttcgtgttg ccacttccag
541 ccgggttcaa atcagagttt tggcttgtgg gttcgtctta acaggcggcc gtggaggtga
601 tacgaaataa tgaaccgttg tctgctgctt aacctgtctc accgttctgg tgaagattcg
661 ttccccgcac tctgcatctc tgctttgcat acctgccgat gttataccca tctcggcgct
721 tctcaggatt caagagctgg ttacagttac tgaggactga acaagggcgc tcttgtaaaa
781 acaagagttt tctcgtggtt tcgccgaact ttcacactta cgttcggtta tgtgcttaat
841 aatgttatga aaaagaaacc ggttgcgcag ttggagcgtc agcattcact gctggaaaat
901 ccatgtgctt atgggttgtt atcgcagttc caggctgcga tagtcgttaa ctgttttaca
961 cttaataaaa taatttgagg ttcgctatgt ctgacgacat gtctatgggt ttgccttcgt
1021 cagcgggcga acacggtgta ctacgctcca tgcaggaggt tgcaatgagc tcccaggaag
1081 ccagcaagat gctgcgtact tacaatattg cctggtgggg caataactac tatgacgtta
1141 acgagctggg ccacattagc gtgtgcccgg acccggacgt cccggaagct cgcgtcgatc
1201 tcgcgcagtt agtgaaaact cgtgaagcac agggccagcg tctgcctgca ctgttctgtt
1261 tcccacagat cctgcagcac cgtttgcgtt ccattaacgc cgcgttcaaa cgtgcgaggg
1321 aatcctacgg ctataacggc gattacttcc ttgtttatcc gatcaaagtt aaccagcacc
1381 gccgcgtgat tgagtccctg attcattcgg gcgaaccgct gggtctggaa gccggttcca
1441 aagccgagtt gatggcagta ctggcacatg ctggcatgac ccgtagcgtc atcgtctgca
1501 acggttataa agaccgcgaa tatatccgcc tggcattaat tggcgagaag atggggcaca
1561 aggtctatct ggtcattgag aagatgtcag aaatcgccat tgtgctggat gaagcagaac
1621 gtctgaatgt cgttcctcgt ctgggcgtgc gtgcacgtct gcgttcgcag ggttcgggta
1681 aatggcagtc ctccggcggg gaaaaatcga agttcggcct ggctgcgact caggtactgc
1741 aactggttga aaccctgcgt gaagccgggc gtctcgacag cctgcaacta ctgcacttcc
1801 acctcggttc gcagatggcg aatattcgcg atatcgcgac aggcgttcgt gaatccgcgc
1861 gtttctatgt ggaactgcac aagctgggcg tcaatattca gtgcttcgac gtcggcggcg
1921 gtctgggcgt ggattatgaa ggtactcgtt cgcagtccga ctgttcggtg aactacggcc
1981 tcaatgaata cgccaacaac attatctggg cgattggcga tgcgtgtgaa gaaaacggtc
2041 tgccgcatcc gacggtaatc accgaatcgg gtcgtgcggt gactgcgcat cacaccgtgc
2101 tggtgtctaa tatcatcggc gtggaacgta acgaatacac ggtgccgacc gcgcctgcag
2161 aagatgcgcc gcgcgcgctg caaagcatgt gggaaacctg gcaggagatg cacgaaccgg
2221 gaactcgccg ttctctgcgt gaatggttac acgacagtca gatggatctg cacgacattc
2281 atatcggcta ctcttccggc atctttagcc tgcaagaacg tgcatgggct gagcagcttt
2341 atttgagcat gtgccatgaa gtgcaaaagc agctggatcc gcaaaaccgt gctcatcgtc
2401 cgattatcga cgagctgcag gaacgtatgg cggacaaaat gtacgtcaac ttctcgctgt
2461 tccagtcgat gccggacgca tgggggatcg accagttgtt cccggttctg ccgctggaag
2521 ggctggatca agtgccggaa cgtcgcgctg tgctgctgga tattacctgt gactctgacg
2581 gtgctatcga ccactatatt gatggtgacg gtattgccac gacaatgcca atgccggagt

2641 acgatccaga gaatccgccg atgctcggtt tctttatggt cggcgcatat caggagatcc
2701 tcggcaacat gcacaacctg ttcggtgata ccgaagcggt tgacgtgttc gtcttccctg
2761 acggtagcgt agaagtagaa ctgtctgacg aaggcgatac cgtggcggac atgctgcaat
2821 atgtacagct cgatccgaaa acgctgttaa cccagttccg cgatcaagtg aagaaaaccg
2881 atcttgatgc tgaactgcaa caacagttcc ttgaagagtt cgaggcaggt ttgtacggtt
2941 atacttatct tgaagatgag taagtcctgt gttacttgaa tccgcttaat ttagcggtga
3001 taatccgcca caatttattg tgacaaatcc aacccttcct cgtcgggcct aacgacgcgg
3061 aagggttttt ttatatcgac tttgtaatag gagtccatcc atgagcacct taggtcatca
3121 atacgataac tcactggttt ccaatgcctt tggtttttta cgcctgccga tgaacttcca
3181 gccgtatgac agcgatgcag actgggtgat tactggcgtg ccgttcgata tggcca

## Claims

1. A DNA plasmid comprising a kanamycin resistance gene comprising a kanamycin resistance gene promoter wherein the kanamycin resistance gene promoter consists of the P1 promoter of Figure 1, wherein the expression of the kanamycin resistance gene is decreased in said DNA plasmid as compared to expression of the kanamycin resistance gene in the plasmid pVR1012 and wherein the decreased kanamycin resistance gene expression results in higher plasmid yields and higher plasmid stability.

2. The DNA plasmid of claim 1 wherein;
(a) the kanamycin resistance gene comprises no promoter other than the P1 promoter of Figure 1, a kanamycin resistance gene coding sequence beginning with an ATG initiator codon, and a rrnB T1-T2 transcription terminator;
(b) the kanamycin resistance gene comprises no promoter other than the P 1 promoter of Figure 1, a kanamycin resistance gene coding sequence beginning with an ATG initiator codon, and no transcription terminator;
(c) the kanamycin resistance gene comprises no promoter other than the P 1 promoter of Figure 1, a kanamycin resistance gene coding sequence beginning with a TTG initiator codon, and a rrnB T1-T2 transcription terminator;
(d) the kanamycin resistance gene comprises no promoter other than the P1 promoter of Figure 1, a kanamycin resistance gene coding sequence beginning with an ATG initiator codon, a rrnB T1-T2 transcription terminator, and wherein the plasmid further comprises a feline immunodeficiency virus (FIV) env gene; or
(e) the kanamycin resistance gene comprises no promoter other than the P1 promoter of Figure 1, a kanamycin resistance gene coding sequence beginning with a TTG initiator codon, and no transcription terminator.

3. A formulation for delivery and expression of an antigen, epitope, immunogen, peptide or polypeptide of interest, wherein the formulation comprises the plasmid of claim 1 or 2 and a pharmaceutically or veterinarily acceptable carrier, vehicle or excipient.

4. The formulation of claim 3, wherein the carrier, vehicle or excipient facilitates transfection and/or improves preservation of the vector or protein.

5. The formulation of claim 3 or 4 wherein the antigen, epitope, immunogen, peptide or polypeptide of interest is derived from an avian, bovine, canine, equine, feline or porcine virus or pathogen.

6. The formulation of any one of claims 3 to 5 for use in stimulating an immune response in an animal.

7. The formulation of any one of claims 3 to 5 for use in eliciting an immune response in an animal.

8. The formulation of claim 6 or 7 for use wherein the animal is an avian, a bovine, a canine, an equine, a feline or a porcine.

9. A kit for stimulating an immune response in an animal or eliciting an immune response in an animal wherein said kit comprises the DNA plasmid of claim 1 or 2 or the formulation of any one of claims 3 to 5 and instructions for stimulating an immune response in an animal or eliciting an immune response in an animal.

## Patentansprüche

1. DNA-Plasmid, umfassend ein Kanamycin-Resistenzgen, das einen Kanamycin-Resistenzgen-Promotor umfasst, wobei der Kanamycin-Resistenzgen-Promotor aus dem P1-Promotor von Figur 1 besteht, wobei die Expression des Kanamycin-Resistenzgens in dem DNA-Plasmid im Vergleich zu der Expression des Kanamycin-Resistenzgens im Plasmid pVR1012 vermindert ist und wobei das Resultat der verminderten Kanamycin-Resistenzgen-Expression ein höherer Plasmidertrag und eine höhere Plasmidstabilität ist.

2. DNA-Plasmid gemäß Anspruch 1, wobei
(a) das Kanamycin-Resistenzgen keinen anderen Promotor als den P1-Promotor von Figur 1, eine das Kanamycin-Resistenzgen codierende Sequenz, die mit einem ATG-Initiatorcodon beginnt, und einen rrnB-T1-T2-Transkriptionsterminator umfasst;
(b) das Kanamycin-Resistenzgen keinen anderen Promotor als den P1-Promotor von Figur 1, eine das Kanamycin-Resistenzgen codierende Sequenz, die mit einem ATG-Initiatorcodon beginnt, und keinen Transkriptionsterminator umfasst;
(c) das Kanamycin-Resistenzgen keinen anderen Promotor als den P1-Promotor von Figur 1, eine das Kanamycin-Resistenzgen codierende Sequenz, die mit einem TTG-Initiatorcodon beginnt, und einen rrnB-T1-T2-Transkriptionsterminator umfasst;
(d) das Kanamycin-Resistenzgen keinen anderen Promotor als den P1-Promotor von Figur 1, eine das Kanamycin-Resistenzgen codierende Sequenz, die mit einem ATG-Initiatorcodon beginnt, und einen rrnB-T1-T2-Transkriptionsterminator umfasst, und wobei das Plasmid des Weiteren ein Feline Immunschwäche-Virus (FIV)-env-Gen umfasst; oder
(e) das Kanamycin-Resistenzgen keinen anderen Promotor als den P1-Promotor von Figur 1, eine das Kanamycin-Resistenzgen codierende Sequenz, die mit einem TTG-Initiatorcodon beginnt, und keinen Transkriptionsterminator umfasst.

3. Formulierung für die Abgabe und Expression eines Antigens, Epitops, Immunogens, Peptids oder Polypeptids von Interesse, wobei die Formulierung das Plasmid gemäß Anspruch 1 oder 2 und ein(en) pharmazeutisch oder veterinärmedizinisch verträglichen Träger, verträgliches Vehikel oder verträglichen Exzipienten umfasst.

4. Formulierung gemäß Anspruch 3, wobei der Träger, das Vehikel oder der Exzipient die Transfektion erleichtert und/oder die Konservierung des Vektors oder Proteins verbessert.

5. Formulierung gemäß Anspruch 3 oder 4, wobei das Antigen, Epitop, Immunogen, Peptid oder Polypeptid von Interesse von einem Vogel-, Rinder-, Hunde-, Pferde-, Katzen- oder Schweine-Virus oder -Pathogen abstammt.

6. Formulierung gemäß einem der Ansprüche 3 bis 5 für die Verwendung zum Stimulieren einer Immunantwort in einem Tier.

7. Formulierung gemäß einem der Ansprüche 3 bis 5 für die Verwendung zum Auslösen einer Immunantwort in einem Tier.

8. Formulierung gemäß Anspruch 6 oder 7 für die Verwendung, bei der das Tier ein Vogel, ein Rind, ein Hund, ein Pferd, eine Katze oder ein Schwein ist.

9. Kit zum Stimulieren einer Immunantwort in einem Tier oder zum Auslösen einer Immunantwort in einem Tier, wobei das Kit das DNA-Plasmid gemäß Anspruch 1 oder 2 oder die Formulierung gemäß einem der Ansprüche 3 bis 5 und eine Anleitung zum Stimulieren einer Immunantwort in einem Tier oder zum Auslösen einer Immunantwort in einem Tier umfasst.

## Revendications

1. Plasmide d'ADN comprenant un gène de résistance à la kanamycine comprenant un promoteur du gène de résistance à la kanamycine, où le promoteur du gène de résistance à la kanamycine est constitué du promoteur P1 de la Figure 1, où l'expression du gène de résistance à la kanamycine est diminuée dans ledit plasmide d'ADN par comparaison à l'expression du gène de résistance à la kanamycine dans le plasmide pVR1012 et où la diminution de l'expression du gène de résistance à la kanamycine entraîne une augmentation des rendements en plasmide et une augmentation de la stabilité du plasmide.

2. Plasmide d'ADN selon la revendication 1, dans lequel :
(a) le gène de résistance à la kanamycine ne comprend pas d'autre promoteur que le promoteur P1 de la Figure 1, et comprend une séquence de codage du gène de résistance à la kanamycine commençant par un codon d'initiation ATG et un terminateur de transcription T1-T2 de *rrnB*;
(b) le gène de résistance à la kanamycine ne comprend pas d'autre promoteur que le promoteur P1 de la Figure 1, comprend une séquence de codage du gène de résistance à la kanamycine commençant par un codon d'initiation ATG, et ne comprend pas de terminateur de transcription;
(c) le gène de résistance à la kanamycine ne comprend pas d'autre promoteur que le promoteur P1 de la Figure 1, et comprend une séquence de codage du gène de résistance à la kanamycine commençant par un codon d'initiation TTG et un terminateur de transcription T1-T2 de rrnB;
(d) le gène de résistance à la kanamycine ne comprend pas d'autre promoteur que le promoteur P1 de la Figure 1, comprend une séquence de codage du gène de résistance à la kanamycine commençant par un codon d'initiation ATG, un terminateur de transcription T1-T2 de rrnB, et où le plasmide comprend en outre un gène *env* du virus d'immunodéficience féline (FIV); ou
(e) le gène de résistance à la kanamycine ne comprend pas d'autre promoteur que le promoteur P1 de la Figure 1, comprend une séquence de codage du gène de résistance à la kanamycine commençant par un codon d'initiation TTG, et ne comprend pas de terminateur de transcription.

3. Formulation destinée à la délivrance et l'expression d'un antigène, d'un épitope, d'un immunogène, d'un peptide ou d'un polypeptide d'intérêt, la formulation comprenant le plasmide de la revendication 1 ou 2 et un support, véhicule ou excipient acceptable sur le plan pharmaceutique ou vétérinaire.

4. Formulation selon la revendication 3, dans laquelle le support, véhicule ou excipient facilite la transfection et/ou améliore la conservation du vecteur ou de la protéine.

5. Formulation selon la revendication 3 ou 4, dans laquelle l'antigène, l'épitope, l'immunogène, le peptide ou le polypeptide d'intérêt provient d'un virus ou d'un pathogène aviaire, bovin, canin, équin, félin ou porcin.

6. Formulation selon l'une quelconque des revendications 3 à 5, à utiliser dans la stimulation d'une réponse immunitaire chez un animal.

7. Formulation selon l'une quelconque des revendications 3 à 5, à utiliser dans le déclenchement d'une réponse immunitaire chez un animal.

8. Formulation selon la revendication 6 ou 7, pour une utilisation dans laquelle l'animal est un oiseau, un bovin, un canin, un équin, un félin ou un porcin.

9. Kit pour la stimulation d'une réponse immunitaire chez un animal ou le déclenchement d'une réponse immunitaire chez un animal, ledit kit comprenant le plasmide d'ADN de la revendication 1 ou 2 ou la formulation de l'une quelconque des revendications 3 à 5 et des instructions pour la stimulation d'une réponse immunitaire chez un animal ou le déclenchement d'une réponse immunitaire chez un animal.
